(19) Europäisches Patentamt
European Patent Office
Office européen des brevets

(11) **EP 3 605 103 A1**

(12) **EUROPEAN PATENT APPLICATION**
published in accordance with Art. 153(4) EPC

(43) Date of publication:
**05.02.2020 Bulletin 2020/06**

(21) Application number: **18777198.5**

(22) Date of filing: **29.03.2018**

(51) Int Cl.:
*G01N 33/68* (2006.01)    *A61K 31/282* (2006.01)
*A61K 31/513* (2006.01)    *A61K 31/519* (2006.01)
*A61K 39/395* (2006.01)    *A61P 35/00* (2006.01)
*G01N 33/50* (2006.01)

(86) International application number:
**PCT/JP2018/013340**

(87) International publication number:
**WO 2018/181759 (04.10.2018 Gazette 2018/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.03.2017 JP 2017070541**

(71) Applicants:
• **Keio University**
**Tokyo 108-8345 (JP)**
• **Kabushiki Kaisha Yakult Honsha**
**Tokyo 105-8660 (JP)**

(72) Inventors:
• **SUGIMOTO, Shinji**
**Tokyo 160-8582 (JP)**
• **TANIGAWARA, Yusuke**
**Tokyo 160-8582 (JP)**
• **MATSUO, Mitsuhisa**
**Tokyo 105-8660 (JP)**
• **TAKAHASHI, Hiroyuki**
**Tokyo 105-8660 (JP)**

(74) Representative: **Blodig, Wolfgang**
**Wächtershäuser & Hartz**
**Patentanwaltspartnerschaft mbB**
**Weinstrasse 8**
**80333 München (DE)**

(54) **COMBINED ANTICANCER AGENT SENSITIVITY DETERMINATION MARKER**

(57) Provided is a novel marker for determining sensitivity to an anti-cancer agent. The marker for determining sensitivity to an anti-cancer agent, the anti-cancer agent including oxaliplatin or a salt thereof, fluorouracil or a salt thereof, and levofolinate or a salt thereof, the marker comprising one or more substances selected from the group consisting of 2DG6P, 2MSE, CSSG, DOPM, GSSG, I4A, P2CB, 1-methyl-2-pyrrolidone, ASP, benzamide, glucaric acid, GL6P, Gly-Gly, HYPT and HYPX.

[Figure 1]

**EP 3 605 103 A1**

**Description**

Technical Field

The present invention relates to a marker for determining sensitivity to an anti-cancer agent which is used to determine whether or not a cancer of a patient of interest has a therapeutic response to the anti-cancer agent. Further, the present invention relates to use of the marker.

Background Art

Anti-cancer agents include various types such as an alkylating agent, a platinum agent, an antimetabolite an anti-cancer antibiotic, and an anti-cancer plant alkaloid. These anti-cancer agents are effective for some cancers but not effective for other cancers. It is known that even when an anti-cancer agent has been confirmed to be effective for a certain cancer, the anti-cancer agent is effective for some patients and not effective for other patients, leading to interindividual differences. Whether or not a cancer of a specific patient has a response to an anti-cancer agent is designated as sensitivity to the anti-cancer agent.

Oxaliplatin, (SP-4-2)-[(1R,2R)-cyclohexane-1,2-diamine-κN,κN'][ethanedioato(2-)-κO$^1$,κO$^2$]platinum (IUPAC), is a third generation platinum-based complex anti-neoplastic agent. The action mechanism thereof is thought to be based on inhibition of DNA synthesis or protein synthesis via cross-linking with DNA bases, similar to precedent platinum-based complex anti-cancer agents such as cisplatin (CDDP) and carboplatin (CBDCA). However, oxaliplatin (L-OHP) exhibits an anti-tumor effect even against colorectal cancer, while CDDP or CBDCA does not, thus oxaliplatin shows different anti-tumor spectrum from the precedent platinum-based complex anti-neoplastic agents. In the United States, oxaliplatin, in combination with fluorouracil (5-FU)/levofolinate (LV), was approved as a first line therapy for metastatic colorectal cancer in January, 2004. In Japan, oxaliplatin was listed in the National Health Insurance price list in April, 2005 for "advanced/recurrent colorectal cancer not amenable to curative surgical resection", as a combination with other anti-neoplastic agents (the usefulness is acknowledged in the case of combination of oxaliplatin with continuous intravenous infusion of levofolinate and fluorouracil and the like). For advanced/recurrent colorectal cancer, 5-FU/LV regimen, which had been employed until early 1990's, provided a survival of 10 to 12 months. In contrast, FOLFOX regimen, which further comprises oxaliplatin, has achieved a survival period of 19.5 months which is almost twice of that of the 5-FU/LV regimen. In August, 2009, the combined use of oxaliplatin with continuous intravenous infusion of levofolinate/fluorouracil was also listed for "postoperative adjuvant chemotherapy for colon cancer", which was added as another efficacy and effectiveness. Thus, oxaliplatin is a drug that is promising for extended use and benefits in colorectal cancer patients. Besides colorectal cancer, the efficacy and effectiveness were added for unresectable pancreatic cancer in August, 2009 and for gastric cancer in March, 2015, as a combination of oxaliplatin with other chemotherapeutic agents.

However, the response rate of FOLFOX regimen against advanced recurrent colorectal cancer is still about 50%. In other words, a half of the patients treated with the regimen fail to obtain the effects. In addition, the use of oxaliplatin may cause neutropenia and peripheral neuropathy in high frequency. These are not lethal side effects but become factors that cause difficulty in continuing the therapy. If there are biomarkers that can predict, before starting the therapy, whether the therapy is effective for a patient (responder) or ineffective for a patient (non-responder), and can diagnose therapeutic response in an early stage of the therapy, it is possible to realize a highly effective and safe chemotherapy.

Furthermore, a therapy schedule of cancer chemotherapy generally requires a long period of time. By monitoring sensitivity to an anti-cancer agent during the therapy over time, it is possible to determine whether or not the therapy should continue. Such a determination leads to reduction of the patient's burden and adverse effects, and may also be beneficial from the viewpoint of medical economics. To realize a "personalized therapy" which predicts a therapeutic response of individual patient and diagnoses in an early stage to select an appropriate agent or therapeutic regimen, it is urgently needed to establish a biomarker which enables to predict an effect of an anti-cancer agent such as oxaliplatin or to diagnose therapeutic response in an early stage of a therapy.

From such viewpoints, the present inventors conducted a comprehensive analysis of intracellular metabolic variation in multiple human cancer cell lines having different drug sensitivity or in cancer-bearing mice transplanted with the human cancer cell lines after they had been exposed to drugs, using capillary electrophoresis time-of-flight mass spectrometer (CE-TOF MS). Then, the inventors conducted a comparative analysis of the results with drug sensitivity to search for a marker for determining sensitivity to an anti-cancer agent, and reported the obtained several markers (Patent Literatures 1 to 4). However, these markers have not yet been put into practical use. Furthermore, combination therapies of FOLFOX regimen with an antibody medicine such as bevacizumab, cetuximab or panitumumab have been recently established. Thus, there is an increasing importance of a biomarker which enables to predict an effect of FOLFOX regimen or to diagnose a therapeutic response in an early stage of the therapy.

Citation List

Patent Literature

Patent Literature 1: WO 2009/096189
Patent Literature 2: WO 2011/052750
Patent Literature 3: WO 2012/127984
Patent Literature 4: WO 2013/125675

Summary of the Invention

Problems to be Solved by the Invention

An object of the present invention is to provide a marker for determining sensitivity to an anti-cancer agent which can identify a therapeutic response of an individual patient. Another object of the present invention is to provide a novel cancer therapeutic means using the marker.

Means for Solving the Problems

In view of the foregoing, the present inventors have conducted a comprehensive analysis of metabolites in blood samples from colorectal cancer patients before starting mFOLFOX6 therapy combined with bevacizumab, using CE-Q-TOF MS and CE-TOF MS. As a result, the inventors have found that the concentrations of 2-deoxyglucose 6-phosphate (2DG6P), 2-methylserine (2MSE), cysteine-glutathione disulphide (CSSG), dopamine (DOPM), oxidized glutathione (GSSG), imidazole-4-acetate (I4A) and pyridine-2-carboxylic acid butyl ester (P2CB) were higher in patients of a responder group whose therapeutic response to the combination therapy of mFOLFOX6 therapy with bevacizumab is high than those in patients of a non-responder group whose therapeutic response to the combination therapy is low. Furthermore, the inventors have found that the concentrations of 1-methyl-2-pyrrolidone, aspartate (ASP), benzamide, glucaric acid, glucose 6-phosphate (GL6P), glycylglycin (Gly-Gly), hypotaurine (HYPT) and hypoxanthine (HYPX) were lower in patients of the responder group than in patients of the non-responder group.

Based on these findings and further studies, the inventors have also found that whether or not a cancer of a cancer patient has sensitivity to an anti-cancer agent can be determined by measuring a concentration of one or more substances selected from the group consisting of 2DG6P, 2MSE, CSSG, DOPM, GSSG, I4A, P2CB, 1-methyl-2-pyrrolidone, ASP, benzamide, glucaric acid, GL6P, Gly-Gly, HYPT and HYPX in a biological sample from the cancer patient. The inventors have also found that, by measuring (1) concentrations of 2DG6P, CSSG, HYPT, I4A and P2CB, (2) concentrations of 2DG6P, 2MSE, ASP, CSSG, DOPM, GL6P and HYPT, or (3) concentrations of CSSG, DOPM and HYPT; digitizing the result of the measuring based on whether the concentration is equal to or more than (or equal to or less than) a cut-off value for responder; and assigning the digitized value to a specific calculation formula, it is possible to determine whether or not a cancer of the cancer patient has sensitivity to an anti-cancer agent, specifically whether or not the cancer patient is a responder. Furthermore, the inventors have found that, by measuring a concentration of one or more substances selected from the group consisting of ASP and CSSG in a biological sample from a cancer patient, it is possible to predict a total tumor diameter before starting the therapy. The inventors have also found that, by measuring a concentration of one or more substances selected from the group consisting of 2-aminobutyric acid, CSSG, gamma-glutamylcysteine (gamma-Glu-Cys), glycerol-3-phosphate, quinic acid, ASP, glycocholic acid, HYPX and lactic acid in a biological sample from a cancer patient, it is possible to predict prognosis in a therapy with an anti-cancer agent.

Furthermore, the inventors have found that, by employing an expression variation of one or more substances selected from the group consisting of 2DG6P, 2MSE, CSSG, DOPM, GSSG, I4A, P2CB, 1-methyl-2-pyrrolidone, ASP, benzamide, glucaric acid, GL6P, Gly-Gly, HYPT, HYPX, 2-aminobutyric acid, gamma-Glu-Cys, glycerol-3-phosphate, quinic acid, glycocholic acid and lactic acid in a biological sample from a cancer patient as an index, screening of an anti-cancer agent sensitivity enhancer can be accomplished. The inventors have also found that, by combining the anti-cancer agent sensitivity enhancer with the anti-cancer agent to be a target of sensitivity enhancement, the therapeutic effect of the anti-cancer agent can be remarkably improved. Consequently, the inventors have completed the present invention.

Accordingly, the present invention provides the following [1] to [22].

[1] A marker for determining sensitivity to an anti-cancer agent, the anti-cancer agent including oxaliplatin or a salt thereof, fluorouracil or a salt thereof, and levofolinate or a salt thereof, the marker comprising one or more substances selected from the group consisting of 2DG6P, 2MSE, CSSG, DOPM, GSSG, I4A, P2CB, 1-methyl-2-pyrrolidone, ASP, benzamide, glucaric acid, GL6P, Gly-Gly, HYPT and HYPX.

[2] The marker for determining sensitivity to an anti-cancer agent according to [1], wherein the anti-cancer agent further includes bevacizumab.

[3] A method for determining sensitivity to an anti-cancer agent, the anti-cancer agent including oxaliplatin or a salt thereof, fluorouracil or a salt thereof, and levofolinate or a salt thereof, the method comprising measuring an amount of one or more substances selected from the group consisting of 2DG6P, 2MSE, CSSG, DOPM, GSSG, I4A, P2CB, 1-methyl-2-pyrrolidone, ASP, benzamide, glucaric acid, GL6P, Gly-Gly, HYPT and HYPX in a biological sample from a cancer patient.

[4] The method for determining sensitivity according to [3], further comprising determining sensitivity of the cancer patient to the anti-cancer agent by comparing a result of the measuring to a control level.

[5] The method for determining sensitivity according to [4], wherein the control level is a cut-off value for a responder, and the cut-off for 2DG6P is $5.304 \times 10^{-4} \leq$; the cut-off for 2MSE is $1.404 \times 10^{-3} \leq$; the cut-off for CSSG is $2.223 \times 10^{-2} \leq$; the cut-off for DOPM is $1.153 \times 10^{-3} \leq$; the cut-off for GSSG is $1.061 \times 10^{-3} \leq$; the cut-off for I4A is $3.316 \times 10^{-3} \leq$; the cut-off for P2CB is $5.952 \times 10^{-4} \leq$; the cut-off for 1-methyl-2-pyrrolidone is $\leq 8.422 \times 10^{-2}$; the cut-off for ASP is $\leq 3.401 \times 10^{-2}$; the cut-off for benzamide is $\leq 9.859 \times 10^{-2}$; the cut-off for glucaric acid is $\leq 1.058 \times 10^{-3}$; the cut-off for GL6P is $\leq 8.167 \times 10^{-4}$; the cut-off for Gly-Gly is $\leq 5.349 \times 10^{-3}$; the cut-off for HYPT is $\leq 1.837 \times 10^{-2}$; and the cut-off for HYPX is $\leq 1.050 \times 10^{-1}$.

[6] The method for determining sensitivity according to [3], further comprising determining whether the cancer patient is a responder or not by calculating probability (p) of the cancer patient being the responder with formula (1):

$$p = \frac{1}{1 + e^{-\left(-3.067 + 19.971 \times (2DG6P) + 2.844 \times (CSSG) + 2.864 \times (HYPT) + 17.68 \times (I4A) + 17.81 \times (P2CB)\right)}} \qquad (1)$$

(wherein, 2DG6P, CSSG, I4A and P2CB represent 1, respectively, when a result of the measuring for each substance is equal to or more than a respective cut-off value, and represent 0, respectively, when the result of the measuring for each substance is less than the respective cut-off value; and HYPT represents 1 when a result of the measuring for HYPT is equal to or less than a cut-off value, and represents 0 when the result of the measuring is more than the cut-off value, and wherein the cut-off for 2DG6P is $5.304 \times 10^{-4}$; the cut-off for CSSG is $2.223 \times 10^{-2}$; the cut-off for I4A is $3.316 \times 10^{-3}$; the cut-off for P2CB is $5.952 \times 10^{-4}$; and the cut-off for HYPT is $1.837 \times 10^{-2}$).

[7] The method for determining sensitivity according to [3], further comprising determining whether the cancer patient is a responder or not by calculating probability (p) of the cancer patient being the responder with formula (2):

$$p = \frac{1}{1 + e^{(-11.5959 - ((2DG6P) + (2MSE) + (ASP) + (CSSG) + (DOPM) + (GL6P) + (HYPT)))}} \qquad (2)$$

(wherein, 2DG6P represents 10.2190 when a result of the measuring for 2DG6P is equal to or more than a cut-off value, and represents -10.2190 when the result of the measuring is less than the cut-off value; 2MSE represents 1.4778 when a result of the measuring for 2MSE is equal to or more than a cut-off value, and represents -1.4778 when the result of the measuring is less than the cut-off value; ASP represents -1.4976 when a result of the measuring for ASP is equal to or more than a cut-off value, and represents 1.4976 when the result of the measuring is less than the cut-off value; CSSG represents 2.0937 when a result of the measuring for CSSG is equal to or more than a cut-off value, and represents -2.0937 when the result of the measuring is less than the cut-off value; DOPM represents 2.2258 when a result of the measuring for DOPM is equal to or more than a cut-off value, and represents -2.2258 when the result of the measuring is less than the cut-off value; GL6P represents -1.6623 when a result of the measuring for GL6P is equal to or more than a cut-off value, and represents 1.6623 when the result is less than the cut-off value; and HYPT represents -2.3200 when a result of the measuring for HYPT is equal to or more than a cut-off value, and represents 2.3200 when the result of the measuring is less than the cut-off value, and wherein the cut-off for 2DG6P is $5.304 \times 10^{-4}$; the cut-off for 2MSE is $1.404 \times 10^{-3}$; the cut-off for ASP is $3.401 \times 10^{-2}$; the cut-off for CSSG is $2.223 \times 10^{-2}$; the cut-off for DOPM is $1.153 \times 10^{-3}$; the cut-off for GL6P is $8.167 \times 10^{-4}$; and the cut-off for HYPT is $1.837 \times 10^{-2}$).

[8] The method for determining sensitivity according to [3], further comprising determining whether the cancer patient is a responder or not by calculating probability (p) of the cancer patient being the responder with formula (3):

$$p = \frac{1}{1+e^{(-1.7898-((CSSG)+(DOPM)+(HYPT)))}} \qquad (3)$$

(wherein, CSSG represents 1.8701 when a result of the measuring for CSSG is equal to or more than a cut-off value, and represents -1.8701 when the result of the measuring is less than the cut-off value; DOPM represents 1.4081 when a result of the measuring for DOPM is equal to or more than a cut-off value, and represents -1.4081 when the result of the measuring is less than the cut-off value; and HYPT represents -1.0869 when a result of the measuring for HYPT is equal to or more than a cut-off value, and represents 1.0869 when the result of the measuring is less than the cut-off value, and wherein the cut-off for CSSG is $2.223 \times 10^{-2}$; the cut-off for DOPM is $1.153 \times 10^{-3}$; and the cut-off for HYPT is $1.837 \times 10^{-2}$).

[9] The method for determining sensitivity according to any of [3] to [8], wherein the biological sample is a biological sample from a cancer patient to whom the anti-cancer agent has been administered.

[10] The method for determining sensitivity according to any of [3] to [9], wherein the anti-cancer agent further includes bevacizumab.

[11] A method for determining a total tumor diameter of a cancer patient, the method comprising measuring an amount of one or more substances selected from the group consisting of ASP and CSSG in a biological sample from the cancer patient.

[12] A marker for predicting prognosis in a therapy with an anti-cancer agent, the anti-cancer agent including oxaliplatin or a salt thereof, fluorouracil or a salt thereof, and levofolinate or a salt thereof, the marker comprising one or more substances selected from the group consisting of 2-aminobutyric acid, CSSG, gamma-Glu-Cys, glycerol-3-phosphate, quinic acid, ASP, glycocholic acid, HYPX and lactic acid.

[13] The marker for predicting prognosis according to [12], wherein the anti-cancer agent further includes bevacizumab.

[14] A method for predicting prognosis in a therapy with an anti-cancer agent, the anti-cancer agent including oxaliplatin or a salt thereof, fluorouracil or a salt thereof, and levofolinate or a salt thereof, the method comprising measuring an amount of one or more substances selected from the group consisting of 2-aminobutyric acid, CSSG, gamma-Glu-Cys, glycerol-3-phosphate, quinic acid, ASP, glycocholic acid, HYPX and lactic acid in a biological sample from a cancer patient.

[15] The method for predicting prognosis according to [14], wherein the anti-cancer agent further includes bevacizumab.

[16] A kit for performing the method for determining sensitivity according to any of [3] to [11], the kit comprising a protocol for measuring an amount of one or more substances selected from the group consisting of 2DG6P, 2MSE, CSSG, DOPM, GSSG, I4A, P2CB, 1-methyl-2-pyrrolidone, ASP, benzamide, glucaric acid, GL6P, Gly-Gly, HYPT and HYPX in the biological sample from the cancer patient.

[17] A kit for performing the method for predicting prognosis according to [14] or [15], the kit comprising a protocol for measuring an amount of one or more substances selected from the group consisting of 2-aminobutyric acid, CSSG, gamma-Glu-Cys, glycerol-3-phosphate, quinic acid, ASP, glycocholic acid, HYPX and lactic acid in the biological sample from the cancer patient.

[18] A screening method for an anti-cancer agent sensitivity enhancer, the anti-cancer agent including oxaliplatin or a salt thereof, fluorouracil or a salt thereof, and levofolinate or a salt thereof, the method comprising employing, as an index, expression variation of one or more substances selected from the group consisting of 2DG6P, 2MSE, CSSG, DOPM, GSSG, I4A, P2CB, 1-methyl-2-pyrrolidone, ASP, benzamide, glucaric acid, GL6P, Gly-Gly, HYPT, HYPX, 2-aminobutyric acid, gamma-Glu-Cys, glycerol-3-phosphate, quinic acid, glycocholic acid and lactic acid in a cancer cell line or a biological sample from a cancer-bearing animal in the presence of the anti-cancer agent.

[19] The screening method according to [18], wherein the anti-cancer agent further includes bevacizumab.

[20] An anti-cancer agent sensitivity enhancer, the anti-cancer agent including oxaliplatin or a salt thereof, fluorouracil or a salt thereof, and levofolinate or a salt thereof, wherein the anti-cancer agent sensitivity enhancer is obtained by the method according to [18] or [19].

[21] A composition for cancer therapy, the composition comprising a combination of the anti-cancer agent sensitivity enhancer according to [20] with an anti-cancer agent including oxaliplatin or a salt thereof, fluorouracil or a salt thereof, and levofolinate or a salt thereof.

[22] The composition for cancer therapy according to [21], wherein the anti-cancer agent further includes bevacizumab.

Effects of the Invention

By use of the marker for determining sensitivity to an anti-cancer agent of the present invention, it becomes possible to determine accurately the sensitivity to an anti-cancer agent, the prognosis or anti-cancer agent resistance of tumor of individual patients before or in an early stage after starting the therapy. As a result, an anti-cancer agent having a higher therapeutic effect can be selected. In addition, since the use of ineffective anti-cancer agents can be avoided, unnecessary adverse effects can be avoided. Furthermore, in a long period therapy schedule with an anti-cancer agent, by determining sensitivity to the anti-cancer agent for each therapy cycle even when the therapy is continuing, it is possible to evaluate the sensitivity of a specific cancer to the anti-cancer agent with time, enabling to determine whether or not the therapy should be further continued. As a result, it is possible to prevent progress of cancer or aggravation of adverse effects associated with the continuous administration of an ineffective anti-cancer agent, leading to mitigation of the patient's burdens and reduction of medical costs.

Furthermore, by using the marker of the present invention, it is possible to select an agent which enhances sensitivity to an anti-cancer agent through screening. By combining the anti-cancer agent sensitivity enhancer with the anti-cancer agent to be a target of sensitivity enhancement, the cancer therapeutic effect can be remarkably improved. Furthermore, a reagent for measuring the marker for determining sensitivity to an anti-cancer agent of the present invention is useful as a reagent for determining sensitivity to an anti-cancer agent.

Brief Description of the Drawings

[Figure 1] Figure 1 shows substances which became significantly different in concentrations between a responder group (R) and a non-responder group (N-R) exhibiting different therapeutic responses to mFOLFOX6 therapy combined with bevacizumab, in analysis method 1.

[Figure 2] Figure 2 shows correlations between a total tumor diameter before starting the therapy and a concentration of ASP or CSSG.

[Figure 3] Figure 3 shows an ROC curve for an anti-cancer agent sensitivity determination model of formula (1).

[Figure 4] Figure 4 shows progression-free survival (PFS, Figure 4A) and overall survival (OS, Figure 4B) in a group of high CSSG concentration (CSSG High) and a group of low CSSG concentration (CSSG Low).

[Figure 5] Figure 5 shows concentration distribution of 9 metabolites among 15 metabolites which became significant in analysis method 2 between the R group and the N-R group exhibiting different therapeutic responses to the mFOLFOX6 therapy combined with bevacizumab.

[Figure 6] Figure 6 shows concentration distribution of 6 metabolites among 15 metabolites which became significant in analysis method 2 between the R group and the N-R group exhibiting different therapeutic responses to the mFOLFOX6 therapy combined with bevacizumab.

[Figure 7] Figure 7 shows (a) an ROC curve for an anti-cancer agent sensitivity determination model of formula (2) (seven metabolites model); and (b) an ROC curve for an anti-cancer agent sensitivity determination model of formula (3) (three metabolites model).

[Figure 8] Figure 8 shows (a) a Kaplan-Meier curve depicted when subjects were divided into the R group and the N-R group in accordance with formula (2) (seven metabolites model) using metabolites before starting the therapy; and (b) a Kaplan-Meier curve depicted when subjects were divided into the R group and the N-R group in accordance with formula (3) (three metabolites model) using metabolites before starting the therapy.

[Figure 9] Figure 9 shows hazard ratios and 95% confidence intervals of metabolites which showed significant differences in the analysis of relationship between metabolite concentration before starting the therapy and OS with the COX proportional hazard model.

[Figure 10] Figure 10 shows Kaplan-Meier curves and the results of Log-rank tests regarding the metabolites having upper limit of 95% confidence interval of less than 1 in the analysis of relationship between metabolite concentration before starting the therapy and OS with the COX proportional hazard model when subjects were divided into groups by cut-off values of the metabolites.

[Figure 11] Figure 11 shows Kaplan-Meier curves and the results of Log-rank tests regarding the metabolites having upper limit of 95% confidence interval of more than 1 in the analysis of relationship between metabolite concentration before starting the therapy and OS with the COX proportional hazard model when subjects were divided into groups by cut-off values of the metabolites.

Modes for Carrying out the Invention

The marker for determining sensitivity to an anti-cancer agent of the present invention comprises any of the following fifteen metabolites: 2-deoxyglucose 6-phosphate (2DG6P), 2-methylserine (2MSE), cysteine-glutathione disulphide (CSSG), dopamine (DOPM), oxidized glutathione (GSSG), imidazole-4-acetate (I4A), pyridine-2-carboxylic acid butyl ester (P2CB), 1-methyl-2-pyrrolidone, aspartate (ASP), benzamide, glucaric acid, glucose 6-phosphate (GL6P), glycylglycin (Gly-Gly), hypotaurine (HYPT) and hypoxanthine (HYPX). As shown in Examples described later, it has been found that among these metabolites, 2DG6P, 2MSE, CSSG, DOPM, GSSG, I4A and P2CB are higher in concentration in patients of the responder group whose therapeutic response to mFOLFOX6 therapy combined with bevacizumab is high than in patients of the non-responder group whose therapeutic response to the combination therapy is low, as a result of comprehensive analysis with CE-Q-TOF MS and CE-TOF MS of the amounts of metabolites in blood before starting mFOLFOX6 therapy combined with bevacizumab using blood samples from colorectal cancer patients. Furthermore, it has been found, as shown in Examples described later, that 1-methyl-2-pyrrolidone, ASP, benzamide, glucaric acid, GL6P, Gly-Gly, HYPT and HYPX are lower in concentration in patients of the responder group than in patients of the non-responder group. Thus, these 15 substances are useful as a marker for determining sensitivity to an anti-cancer agent including oxaliplatin or a salt thereof, fluorouracil or a salt thereof, and levofolinate or a salt thereof, in particular, as a marker for determining sensitivity to an anti-cancer agent including oxaliplatin or a salt thereof, fluorouracil or a salt thereof, levofolinate or a salt thereof, and bevacizumab. Among these substances, (1) a set of 5 substances of 2DG6P, CSSG, HYPT, I4A and P2CB, (2) a set of 7 substances of 2DG6P, 2MSE, ASP, CSSG, DOPM, GL6P and HYPT, or (3) a set of 3 substances of CSSG, DOPM and HYPT are particularly useful as the marker and it becomes possible to determine whether a cancer patient as a subject is a responder or not by using these substances.

Further, as shown in Examples described later, it has been found that ASP is positively correlated with a total tumor diameter of a cancer patient before starting the therapy, and CSSG is negatively correlated with that. Thus, ASP and CSSG are useful as a marker for determining a total tumor diameter of a cancer patient.

Furthermore, as shown in Examples described later, it has been found, from an analysis of progression-free survival (PFS) and overall survival (OS) based on CSSG level, that PFS and OS are significantly longer in CSSG high group than in CSSG low group. Thus, CSSG alone is useful as a marker for predicting prognosis, in particular, as a marker for predicting not only a length of PFS, but also a length of OS in a therapy including the secondary or later therapy with an anti-cancer agent including oxaliplatin or a salt thereof, fluorouracil or a salt thereof, and levofolinate or a salt thereof, in particular with an anti-cancer agent including oxaliplatin or a salt thereof, fluorouracil or a salt thereof, levofolinate or a salt thereof, and bevacizumab.

Furthermore, as shown in Examples described later, it has been found, from the result of analysis by the COX proportional hazard model, that the higher the blood concentrations of 2-aminobutyric acid, CSSG, gamma-glutamyl cysteine (gamma-Glu-Cys), glycerol-3-phosphate and quinic acid, the longer the survival, while the higher the blood concentrations of ASP, glycocholic acid, HYPX and lactic acid, the shorter the survival. Thus, these 9 substances are useful as a marker when used singly for predicting prognosis, in particular, as a marker for predicting a length of OS in a therapy including the secondary or later therapy with an anti-cancer agent including oxaliplatin or a salt thereof, fluorouracil or a salt thereof, and levofolinate or a salt thereof, in particular with an anti-cancer agent including oxaliplatin or a salt thereof, fluorouracil or a salt thereof, levofolinate or a salt thereof, and bevacizumab.

2DG6P is formed by glucose hexokinase from 2-deoxy-D-glucose which has H instead of OH group at the 2-position of glucose after 2-deoxy-D-glucose is taken up by cells. It is known from non-clinical studies that 2DG6P accumulates in the cell due to not being metabolized by the glycolytic pathway, inhibits glucose metabolism due to feedback inhibition of hexokinase, and suppresses growth of cancer cells. However, there have been no reports of studying 2DG6P in serum of cancer patients in a clinical study in cancer patients. So, it is not known at all that 2DG6P can be used as a marker for determining sensitivity to an anti-cancer agent including oxaliplatin or a salt thereof, fluorouracil or a salt thereof, and levofolinate or a salt thereof. It is also not known at all that a high concentration of 2DG6P can determine a responder.

2MSE is a substance which is blended in cosmetics or the like as a moisturizing component. However, it is not known at all that 2MSE can be used as a marker for determining sensitivity to an anti-cancer agent including oxaliplatin or a salt thereof, fluorouracil or a salt thereof, and levofolinate or a salt thereof. It is also not known at all that a high concentration of 2MSE can determine a responder.

CSSG and GSSG are both metabolites of GSH which is known as a substance involved in drug detoxification. CSSG is a complex of GSH and Cys, and GSSG is a dimer of GSH formed by oxidation of GSH. It is known that GSH in blood is rapidly oxidized after blood collection to form GSSG. It is also known that GSH after blood collection is rapidly and more largely bonded to Cys which is more abundant in blood than GSH to form CSSG in a few minutes. However, there have been no reports focusing on CSSG and GSSG with relation to efficacy, including reports associated with cancer. Further, it is not known at all that CSSG or GSSG can be used as a marker for determining sensitivity to an anti-cancer agent including oxaliplatin or a salt thereof, fluorouracil or a salt thereof, and levofolinate or a salt thereof. It is also not known at all that a high concentration of CSSG or GSSG can determine a responder. Furthermore, it is not known at all that CSSG can be used as a marker for predicting prognosis in a therapy with an anti-cancer agent including oxaliplatin or a salt thereof, fluorouracil or a salt thereof, and levofolinate or a salt thereof, particularly as a marker for predicting PFS and OS. It is also not known at all that a high concentration of CSSG indicates the survival is long. In addition, it is also not known at all that CSSG can be used as a marker for predicting a total tumor diameter in vivo.

DOPM is a neurotransmitter present in the central nervous system, and is involved in motor control, hormonal regulation, emotion, motivation, learning, and the like. It is not known at all that DOPM can be used as a marker for determining sensitivity to an anti-cancer agent including oxaliplatin or a salt thereof, fluorouracil or a salt thereof, and levofolinate or a salt thereof. It is also not known at all that a high concentration of DOPM can determine a responder.

I4A is a substance on the metabolic pathway of histamine which is a phlogogenic substance produced by mast cells, basophils and macrophages. It is not known at all that I4A can be used as a marker for determining sensitivity to an anti-cancer agent including oxaliplatin or a salt thereof, fluorouracil or a salt thereof, and levofolinate or a salt thereof. It is also not known at all that a high concentration of I4A can determine a responder.

P2CB is a butyl ester of pyridine-2-carboxylic acid. Pyridine-2-carboxylic acid is a substance on the metabolic pathway of tryptophan. It is known that pyridine-2-carboxylic acid has chelating action, and acts as an activation inhibitor of glucose dehydrogenase. It is also known that picolinic acid treatment inhibits about 50% of protein synthesis in cells. However, it is not known at all that P2CB can be used as a marker for determining sensitivity to an anti-cancer agent including oxaliplatin or a salt thereof, fluorouracil or a salt thereof, and levofolinate or a salt thereof. It is also not known at all that a high concentration of P2CB can determine a responder.

1-Methyl-2-pyrrolidone is known as an intermediate of pharmaceuticals and a synthesis reagent or the like. However, it is not known at all that 1-methyl-2-pyrrolidone can be used as a marker for determining sensitivity to an anti-cancer agent including oxaliplatin or a salt thereof, fluorouracil or a salt thereof, and levofolinate or a salt thereof. It is also not known at all that a high concentration of 1-methyl-2-pyrrolidone can determine a non-responder.

ASP is one of amino acids. In general, it is known that ASP has a central role in nitrogen process in vivo. It has already been known that ASP can be used as a marker for determining sensitivity to an anti-cancer agent including oxaliplatin or a salt thereof and fluorouracil or a salt thereof. It is also known that ASP is higher in concentration in oxaliplatin high-sensitive cell lines than in oxaliplatin low-sensitive cell lines (WO 2013/125675) . However, it is not known at all that ASP can be used as a marker for determining sensitivity to a triple combination anti-cancer agent including further levofolinate or a salt thereof. It is also not known at all that a high concentration of ASP can determine a non-responder. Furthermore, it is not known at all that ASP can be used as a marker for predicting prognosis in a therapy with an anti-cancer agent including oxaliplatin or a salt thereof, fluorouracil or a salt thereof, and levofolinate or a salt thereof. It is also not known at all that a high concentration of ASP indicates the survival is short.

Benzamide is also known as benzoic acid amide, and its derivatives have been used as a sedative and an antipsychotic. However, it is not known at all that benzamide can be used as a marker for determining sensitivity to an anti-cancer agent including oxaliplatin or a salt thereof, fluorouracil or a salt thereof, and levofolinate or a salt thereof. It is also not known at all that a high concentration of benzamide can determine a non-responder.

Glucaric acid is one of typical sugar acids. There is an increasing demand of using glucaric acid as a raw material since its derivatives can be used as a solvent or the like. Several methods for producing them have been known (WO 2013/125509). However, it is not known at all that glucaric acid can be used as a marker for determining sensitivity to an anti-cancer agent including oxaliplatin or a salt thereof, fluorouracil or a salt thereof, and levofolinate or a salt thereof. It is also not known at all that a high concentration of glucaric acid can determine a non-responder.

GL6P is a substance which is metabolized by the pentose phosphate pathway, glycolytic pathway or the like. However, it is not known at all that GL6P can be used as a marker for determining sensitivity to an anti-cancer agent including oxaliplatin or a salt thereof, fluorouracil or a salt thereof, and levofolinate or a salt thereof. It is also not known at all that a high concentration of GL6P can determine a non-responder.

Gly-Gly is used as a buffer for biology experiments and as a starting material for synthesis of more complicated peptide. However, it is not known at all that Gly-Gly can be used as a marker for determining sensitivity to an anti-cancer agent including oxaliplatin or a salt thereof, fluorouracil or a salt thereof, and levofolinate or a salt thereof. It is also not known at all that a high concentration of Gly-Gly can determine a non-responder.

HYPT is an intermediate product in the biosynthesis of taurine from cysteine, and it is synthesized by oxidation of 3-sulfino-L-alanine or cysteamine. It is known that HYPT has an anti-inflammatory action (JP-A-2017-7980) and an antioxidant action (JP-A-2017-14167). However, it is not known at all that HYPT can be used as a marker for determining sensitivity to an anti-cancer agent including oxaliplatin or a salt thereof, fluorouracil or a salt thereof, and levofolinate or a salt thereof. It is also not known at all that a high concentration of HYPT can determine a non-responder.

HYPX is a metabolite on the purine metabolic pathway, and it is synthesized from inosine or xanthine with $H_2O_2$ generated from $O_2^-$ by hydrogen peroxidase. It is known that HYPX can serve as a diagnostic biomarker for osteoarthritis and prostate cancer (JP-A-2006-504093 and JP-A-2016-153808). However, it is not known at all that HYPX can be used as a marker for determining sensitivity to an anti-cancer agent including oxaliplatin or a salt thereof, fluorouracil or a salt thereof, and levofolinate or a salt thereof. It is also not known at all that a high concentration of HYPX can determine a non-responder. Furthermore, it is not known at all that HYPX can be used as a marker for predicting prognosis in a therapy with an anti-cancer agent including oxaliplatin or a salt thereof, fluorouracil or a salt thereof, and levofolinate or a salt thereof. It is also not known at all that a high concentration of HYPX indicates the survival is short.

2-Aminobutyric acid is a metabolite on the cysteine/methionine metabolic pathway. However, it is not known at all that 2-aminobutyric acid can be used as a marker for predicting prognosis in a therapy with an anti-cancer agent including oxaliplatin or a salt thereof, fluorouracil or a salt thereof, and levofolinate or a salt thereof. It is also not known at all that a high concentration of 2-aminobutyric acid indicates the survival is long.

gamma-Glu-Cys is a metabolite on the glutathione metabolic pathway. However, it is not known at all that gamma-Glu-Cys can be used as a marker for predicting prognosis in a therapy with an anti-cancer agent including oxaliplatin or a salt thereof, fluorouracil or a salt thereof, and levofolinate or a salt thereof. It is also not known at all that a high concentration of gamma-Glu-Cys indicates the survival is long.

Glycerol-3-phosphate is a metabolite on the glycerolipid and glycerophosphate metabolic pathways, and the choline metabolic pathway in cancer. However, it is not known at all that glycerol-3-phosphate can be used as a marker for predicting prognosis in a therapy with an anti-cancer agent including oxaliplatin or a salt thereof, fluorouracil or a salt thereof, and levofolinate or a salt thereof. It is also not known at all that a high concentration of glycerol-3-phosphate indicates the survival is long.

Quinic acid is a metabolite on the phenylalanine/ tyrosine/tryptophan synthetic pathway. However, it is not known at all that quinic acid can be used as a marker for predicting prognosis in a therapy with an anti-cancer agent including oxaliplatin or a salt thereof, fluorouracil or a salt thereof, and levofolinate or a salt thereof. It is also not known at all that a high concentration of quinic acid indicates the survival is long.

Glycocholic acid is a metabolite on the bile acid synthetic system and the cholesterol metabolic pathway. However, it is not known at all that glycocholic acid can be used as a marker for predicting prognosis in a therapy with an anti-cancer agent including oxaliplatin or a salt thereof, fluorouracil or a salt thereof, and levofolinate or a salt thereof. It is also not known at all that a high concentration of glycocholic acid indicates the survival is short.

Lactic acid is involved in a variety of pathways as a substance related to glycolysis and gluconeogenesis. However, it is not known at all that lactic acid can be used as a marker for predicting prognosis in a therapy with an anti-cancer agent including oxaliplatin or a salt thereof, fluorouracil or a salt thereof, and levofolinate or a salt thereof. It is also not known at all that a high concentration of lactic acid indicates the survival is short.

As used herein, the responder refers to a patient whose maximum effect during the test therapy period shows a complete response or a partial response as a result of the diagnostic imaging by radiation diagnostician in accordance with RECIST criteria (J Natl Cancer Inst. 2000 Feb 2; 92 (3): 205-16). Furthermore, as used herein, the non-responder (N-R) refers to a patient whose maximum effect during the test therapy period shows stable disease or progressive disease as a result of the diagnostic imaging by radiation diagnostician in accordance with RECIST criteria.

The anti-cancer agent which is a target of the marker for determining sensitivity to an anti-cancer agent of the present invention encompasses not only an anti-cancer agent including oxaliplatin or a salt thereof, fluorouracil or a salt thereof, and levofolinate or a salt thereof, but also an anti-cancer agent which is metabolized in the body and converted into oxaliplatin, fluorouracil, or levofolinate. It has been clarified that tegafur and capecitabine are metabolized in the body, and converted into fluorouracil. Thus, tegafur or capecitabine instead of fluorouracil may also be a target of the marker for determining sensitivity to an anti-cancer agent of the present invention. In that case, an anti-cancer agent including oxaliplatin or a salt thereof, tegafur or a salt thereof, and levofolinate or a salt thereof, and an anti-cancer agent including oxaliplatin or a salt thereof, capecitabine or a salt thereof, and levofolinate or a salt thereof are a target of the marker for determining sensitivity to an anti-cancer agent of the present invention.

Examples of other anti-cancer agents to be used in combination with the anti-cancer agent including oxaliplatin or a salt thereof, fluorouracil or a salt thereof, and levofolinate or a salt thereof include, but are not particularly limited to, cyclophosphamide, ifosfamide, thiotepa, melphalan, busulfan, nimustine, ranimustine, dacarbazine, procarbazine, temozolomide, cisplatin, carboplatin, nedaplatin, methotrexate, pemetrexed, tegaful/uracil, doxifluridine, tegaful/gimeracil/oteracil, capecitabine, cytarabine, enocitabine, gemcitabine, 6-mercaptopurine, fuludarabin, pentostatin, cladribine, hydroxyurea, doxorubicin, epirubicin, daunorubicin, idarubicine, pirarubicin, mitoxantrone, amurubicin, actinomycin D, bleomycin, pepleomycin, mytomycin C, aclarubicin, zinostatin, vincristine, vindesine, vinblastine, vinorelbine, paclitaxel, docetaxel, irinotecan, SN-38, nogitecan, topotecan, etoposide, prednisolone, dexamethasone, tamoxifen, toremifene, medroxyprogesterone, anastrozole, exemestane, letrozole, rituximab, imatinib, gefitinib, gemtuzumab/ozogamicin, bortezomib, erlotinib, cetuximab, bevacizumab, sunitinib, sorafenib, dasatinib, panitumumab, asparaginase, tretinoin, arsenic trioxide, or a salt thereof, or an active metabolite thereof. Among them, irinotecan, SN-38 or a salt thereof, or bevacizumab is preferred, and bevacizumab is particularly preferred.

To determine sensitivity to an anti-cancer agent by employing the marker for determining sensitivity to an anti-cancer agent of the present invention, it may be performed by measuring an amount of one or more substances selected from the group consisting of 2DG6P, 2MSE, CSSG, DOPM, GSSG, I4A, P2CB, 1-methyl-2-pyrrolidone, ASP, benzamide, glucaric acid, GL6P, Gly-Gly, HYPT and HYPX in a biological sample (specimen) derived from a cancer patient. Specifically, it may be further performed by comparing a result of the measuring with a control level. Examples of the control level include a standard concentration, a concentration range in a responder, a cut-off value for a responder (hereinafter, the cut-off value means a relative concentration in the case of the concentration of internal standard solution for LC/MS being 1), and a concentration of the marker for determining sensitivity to an anti-cancer agent of the present invention before administration of the anti-cancer agent. Herein, the cut-off for 2DG6P is $5.304 \times 10^{-4} \leq$; the cut-off for 2MSE is

$1.404 \times 10^{-3} \leq$; the cut-off for CSSG is $2.223 \times 10^{-2} \leq$; the cut-off for DOPM is $1.153 \times 10^{-3} \leq$; the cut-off for GSSG is $1.061 \times 10^{-3} \leq$; the cut-off for I4A is $3.316 \times 10^{-3} \leq$; the cut-off for P2CB is $5.952 \times 10^{-4} \leq$; the cut-off for 1-methyl-2-pyrrolidone is $\leq 8.422 \times 10^{-2}$; the cut-off for ASP is $\leq 3.401 \times 10^{-2}$; the cut-off for benzamide is $\leq 9.859 \times 10^{-2}$; the cut-off for glucaric acid is $\leq 1.058 \times 10^{-3}$; the cut-off for GL6P is $\leq 8.167 \times 10^{-4}$; the cut-off for Gly-Gly is $\leq 5.349 \times 10^{-3}$; the cut-off for HYPT is $\leq 1.837 \times 10^{-2}$; and the cut-off for HYPX is $\leq 1.050 \times 10^{-1}$. Alternatively, it may be performed by measuring (1) the amounts of 2DG6P, CSSG, HYPT, I4A, and P2CB, (2) the amounts of 2DG6P, 2MSE, ASP, CSSG, DOPM, GL6P, and HYPT; or (3) the amounts of CSSG, DOPM, and HYPT in a biological sample (specimen) derived from a cancer patient. Specifically, it may be further performed by comparing a result of the measuring with a cut-off value for each substance for a responder (wherein the cut-off for 2DG6P is $5.304 \times 10^{-4}$; the cut-off for 2MSE is $1.404 \times 10^{-3}$; the cut-off for CSSG is $2.223 \times 10^{-2}$; the cut-off for DOPM is $1.153 \times 10^{-3}$; the cut-off for I4A is $3.316 \times 10^{-3}$; the cut-off for P2CB is $5.952 \times 10^{-4}$; the cut-off for ASP is $3.401 \times 10^{-2}$; the cut-off for GL6P is $8.167 \times 10^{-4}$; and the cut-off for HYPT is $1.837 \times 10^{-2}$) to digitize the result of the measuring, and assigning the digitized value to a specific calculation formula.

Herein, the cancer patient includes a subject who suffers from cancer or who suffered from cancer. Examples of the biological sample include blood, serum, plasma, a cancer tissue biopsy specimen, a cancer extirpation specimen, feces, urine, peritoneal fluid, pleural fluid, cerebrospinal fluid and sputum. Of these, serum is particularly preferred.

Examples of the cancer which the present invention targets include lip, oral and pharyngeal cancers such as pharyngeal cancer; gastrointestinal tract cancers such as esophageal cancer, gastric cancer, and colorectal cancer; respiratory and pleural organ cancers such as lung cancer; bone and articular cartilage cancers; malignant skin melanoma, squamous cell cancer, and other skin cancers; mesothelial and soft tissue cancers such as mesothelioma; female genital cancers such as breast cancer, uterine cancer, and ovarian cancer; male genital cancers such as prostate cancer; urinary tract cancers such as bladder cancer; eye, brain, and central nervous system cancers such as brain tumor; thyroid and other endocrine cancers; lymphoid tissue, hematopoietic tissue, and other related tissue cancers such as non-Hodgkin's lymphoma and lymphoid leukemia; and metastatic cancers from the aforementioned cancers as primary foci. Among them, the present invention is preferably applied to colorectal cancer (large intestine cancer), and particularly preferably to cancer before chemotherapy.

The means for measuring substances selected from the group consisting of 2DG6P, 2MSE, CSSG, DOPM, GSSG, I4A, P2CB, 1-methyl-2-pyrrolidone, ASP, benzamide, glucaric acid, GL6P, Gly-Gly, HYPT, HYPX, 2-aminobutyric acid, gamma-Glu-Cys, glycerol-3-phosphate, quinic acid, glycocholic acid and lactic acid in a specimen can be appropriately determined based on substances to be measured. Examples of the measuring means include various types of mass spectrometers such as CE-Q-TOF MS, CE-TOF MS and gas chromatography-mass spectrometry (GC-MS), HPLC, immunological assays, and biochemical assays.

To determine sensitivity to an anti-cancer agent of interest by employing one or more substances selected from the group consisting of 2DG6P, 2MSE, CSSG, DOPM, GSSG, I4A and P2CB, the amount such as a concentration of one or more substances selected from the group consisting of 2DG6P, 2MSE, CSSG, DOPM, GSSG, I4A and P2CB in a biological sample from a cancer patient may be measured before administration of or in an early stage after administration of the anti-cancer agent. When the measured concentration is evaluated as higher than a predetermined control level, it can be determined that the cancer is sensitive to the anti-cancer agent of interest. Thus, the marker for determining sensitivity to an anti-cancer agent can be used as a marker for actively continuing the therapy to a patient who is expected to receive a therapeutic effect. On the other hand, when the measured concentration is evaluated as lower than a predetermined control level, it can be determined that the cancer is not sensitive to the anti-cancer agent of interest. When the cancer is not sensitive to the anti-cancer agent of interest, a beneficial effect of the anti-cancer agent to the cancer is not expected. If the administration of the anti-cancer agent whose beneficial effect is not expected is carried out or continued, progress of cancer or aggravation of adverse effects would become a concern. Thus, the marker for determining sensitivity to an anti-cancer agent of the present invention can be used not only as a marker for actively continuing the therapy to a patient who is expected to receive a therapeutic effect, but also as a marker for avoiding progress of cancer or aggravation of adverse effects associated with continuous administration of the anti-cancer agent whose beneficial effect is not expected.

Examples of the control level include a cut-off value. Examples of the cut-off value include a cut-off value of $5.304 \times 10^{-4} \leq$ for 2DG6P; a cut-off value of $1.404 \times 10^{-3} \leq$ for 2MSE; a cut-off value of $2.223 \times 10^{-2} \leq$ for CSSG; a cut-off value of $1.153 \times 10^{-3} \leq$ for DOPM; a cut-off value of $1.061 \times 10^{-3} \leq$ for GSSG; a cut-off value of $3.316 \times 10^{-3} \leq$ for I4A; and a cut-off value of $5.952 \times 10^{-4} \leq$ for P2CB.

To determine sensitivity to an anti-cancer agent of interest by employing one or more substances selected from the group consisting of 1-methyl-2-pyrrolidone, ASP, benzamide, glucaric acid, GL6P, Gly-Gly, HYPT and HYPX, the amount such as a concentration of one or more substances selected from the group consisting of 1-methyl-2-pyrrolidone, ASP, benzamide, glucaric acid, GL6P, Gly-Gly, HYPT and HYPX in a biological sample from a cancer patient may be measured before administration of or in an early stage after administration of the anti-cancer agent. When the measured concentration is evaluated as lower than a predetermined control level, it can be determined that the cancer is sensitive to the anti-cancer agent of interest. Thus, the marker for determining sensitivity to an anti-cancer agent can be used as a marker for actively continuing the therapy to a patient who is expected to receive a therapeutic effect. On the other hand, when the measured concentration is evaluated as higher than a predetermined control level, it can be determined that the cancer is not sensitive to the anti-cancer agent of interest. When the cancer is not sensitive to the anti-cancer agent of interest, a beneficial effect of the anti-cancer agent is not expected. If the administration of the anti-cancer agent whose beneficial effect is not expected is carried out or continued, progress of cancer or aggravation of adverse effects would become a concern. Thus, the marker for determining sensitivity to an anti-cancer agent of the present invention can be used not only as a marker for actively continuing the therapy to a patient who is expected to receive a therapeutic effect, but also as a marker for avoiding progress of cancer or aggravation of adverse effects associated with continuous administration of the anti-cancer agent whose beneficial effect is not expected.

Examples of the control level include a cut-off value. Examples of the cut-off value include a cut-off value of $\leq 8.422 \times 10^{-2}$ for 1-methyl-2-pyrrolidone; a cut-off value of $\leq 3.401 \times 10^{-2}$ for ASP; a cut-off value of $\leq 9.859 \times 10^{-2}$ for benzamide; a cut-off value of $\leq 1.058 \times 10^{-3}$ for glucaric acid; a cut-off value of $\leq 8.167 \times 10^{-4}$ for GL6P; a cut-off value of $\leq 5.349 \times 10^{-3}$ for Gly-Gly; a cut-off value of $\leq 1.837 \times 10^{-2}$ for HYPT; and a cut-off value of $\leq 1.050 \times 10^{-1}$ for HYPX.

To determine sensitivity to an anti-cancer agent of interest by employing 2DG6P, CSSG, HYPT, I4A and P2CB, the amounts such as concentrations of 2DG6P, CSSG, HYPT, I4A and P2CB in a biological sample from a cancer patient may be measured before administration of or in an early stage after administration of the anti-cancer agent. Further, regarding 2DG6P, CSSG, I4A and P2CB, when a result of the measuring for each substance is equal to or more than a respective cut-off value, 1 may be assigned to formula (1), and when the result of the measuring for each is less than the respective cut-off value, 0 may be assigned to the formula (1); and when a result of the measuring for HYPT is equal to or less than a cut-off value, 1 may be assigned to formula (1), and when the result of the measuring is more than the cut-off value, 0 may be assigned to formula (1):

$$p = \frac{1}{1+e^{-(-3.067+19.971\times(2DG6P)+2.844\times(CSSG)+2.864\times(HYPT)+17.68\times(I4A)+17.81\times(P2CB))}} \qquad (1)$$

(wherein, 2DG6P, CSSG, I4A and P2CB represent 1 respectively when a result of the measuring for each substance is equal to or more than a respective cut-off value, and represent 0 respectively when a result of the measuring for each is less than the respective cut-off value; and HYPT represents 1 when a result of the measuring for HYPT is equal to or less than a cut-off value, and represents 0 when the result of the measuring for HYPT is more than the cut-off value).

Herein, the cut-off values of the respective substances are as follows: the cut-off for 2DG6P is $5.304 \times 10^{-4}$; the cut-off for CSSG is $2.223 \times 10^{-2}$; the cut-off for HYPT is $1.837 \times 10^{-2}$; the cut-off for I4A is $3.316 \times 10^{-3}$; and the cut-off for P2CB is $5.952 \times 10^{-4}$.

The formula (1) calculates p which represents probability of a cancer patient of interest being a responder. When p is 0.5 or more, it can be determined that the cancer of the cancer patient is sensitive to the anti-cancer agent of interest, that is, the cancer patient is a responder. Thus, the marker for determining sensitivity to an anti-cancer agent can be used as a marker for actively continuing the therapy to a patient who is expected to receive a therapeutic effect. On the other hand, when p is less than 0.5, it can be determined that the cancer of the cancer patient is not sensitive to the anti-cancer agent of interest, that is, the cancer patient is a non-responder. When the cancer is not sensitive to the anti-cancer agent of interest, a beneficial effect of the anti-cancer agent is not expected. If the administration of the anti-cancer agent whose beneficial effect is not expected is carried out or continued, progress of cancer or aggravation of adverse effects would become a concern. Thus, the marker for determining sensitivity to an anti-cancer agent of the present invention can be used not only as a marker for actively continuing the therapy to a patient who is expected to receive a therapeutic effect, but also as a marker for avoiding progress of cancer or aggravation of adverse effects associated with continuous administration of the anti-cancer agent whose beneficial effect is not expected.

To determine sensitivity to an anti-cancer agent of interest by employing 2DG6P, 2MSE, ASP, CSSG, DOPM, GL6P and HYPT, the amounts such as concentrations of 2DG6P, 2MSE, ASP, CSSG, DOPM, GL6P and HYPT in a biological sample from a cancer patient may be measured before administration of or in an early stage after administration of the anti-cancer agent, and then, assignments to formula (2) may be carried out as follows:

$$p = \frac{1}{1+e^{(-11.5959-((2DG6P)+(2MSE)+(ASP)+(CSSG)+(DOPM)+(GL6P)+(HYPT)))}} \qquad (2)$$

(wherein, 2DG6P represents 10.2190 when a result of the measuring for 2DG6P is equal to or more than a cut-off value, and represents -10.2190 when the result of the measuring is less than the cut-off value; 2MSE represents 1.4778 when a result of the measuring for 2MSE is equal to or more than a cut-off value, and represents -1.4778 when the result of the measuring is less than the cut-off value; ASP represents -1.4976 when a result of the measuring for ASP is equal to or more than a cut-off value, and represents 1.4976 when the result of the measuring is less than the cut-off value; CSSG represents 2.0937 when a result of the measuring for CSSG is equal to or more than a cut-off value, and represents -2.0937 when the result of the measuring is less than the cut-off value; DOPM represents 2.2258 when a result of the measuring for DOPM is equal to or more than a cut-off value, and represents -2.2258 when the result of the measuring is less than the cut-off value; GL6P represents -1.6623 when a result of the measuring for GL6P is equal to or more than a cut-off value, and represents 1.6623 when the result is less than the cut-off value; and HYPT represents -2.3200 when a result of the measuring for HYPT is equal to or more than a cut-off value, and represents 2.3200 when the result of the measuring is less than the cut-off value).

Herein, the cut-off values for the respective substance are as follows: the cut-off for 2DG6P is $5.304 \times 10^{-4}$; the cut-off for 2MSE is $1.404 \times 10^{-3}$; the cut-off for ASP is $3.401 \times 10^{-2}$; the cut-off for CSSG is $2.223 \times 10^{-2}$; the cut-off for DOPM is $1.153 \times 10^{-3}$; the cut-off for GL6P is $8.167 \times 10^{-4}$; and the cut-off for HYPT is $1.837 \times 10^{-2}$.

The formula (2) calculates p which represents probability of a cancer patient of interest being a responder. When p is 0.5 or more, it can be determined that the cancer of the cancer patient is sensitive to the anti-cancer agent of interest, that is, the cancer patient is a responder. Thus, the marker for determining sensitivity to an anti-cancer agent can be used as a marker for actively continuing the therapy to a patient who is expected to receive a therapeutic effect. On the other hand, when p is less than 0.5, it can be determined that the cancer of the cancer patient is not sensitive to the anti-cancer agent of interest, that is, the cancer patient is a non-responder. When the cancer is not sensitive to the anti-cancer agent of interest, a beneficial effect of the anti-cancer agent is not expected. If the administration of the anti-cancer agent whose beneficial effect is not expected is carried out or continued, progress of cancer or aggravation of adverse effects would become a concern. Thus, the marker for determining sensitivity to an anti-cancer agent of the present invention can be used not only as a marker for actively continuing the therapy to a patient who is expected to receive a therapeutic effect, but also as a marker for avoiding progress of cancer or aggravation of adverse effects associated with continuous administration of the anti-cancer agent whose beneficial effect is not expected.

To determine sensitivity to an anti-cancer agent of interest by employing CSSG, DOPM and HYPT, the amounts such as concentrations of CSSG, DOPM and HYPT in a biological sample from a cancer patient may be measured before administration of or after administration of the anti-cancer agent, and then, assignments to formula (3) may be carried out as follows:

$$p = \frac{1}{1 + e^{(-1.7898 - ((CSSG) + (DOPM) + (HYPT)))}} \tag{3}$$

(wherein, CSSG represents 1.8701 when a result of the measuring for CSSG is equal to or more than a cut-off value, and represents -1.8701 when the result of the measuring is less than the cut-off value; DOPM represents 1.4081 when a result of the measuring for DOPM is equal to or more than a cut-off value, and represents -1.4081 when the result of the measuring is less than the cut-off value; and HYPT represents -1.0869 when a result of the measuring for HYPT is equal to or more than a cut-off value, and represents 1.0869 when the result of the measuring is less than the cut-off value).

Herein, the cut-off values for the respective substances are as follows: the cut-off for CSSG is $2.223 \times 10^{-2}$; the cut-off for DOPM is $1.153 \times 10^{-3}$; and the cut-off for HYPT is $1.837 \times 10^{-2}$.

The formula (3) calculates p which represents probability of a cancer patient of interest being a responder. When p is 0.5 or more, it can be determined that the cancer of the cancer patient is sensitive to the anti-cancer agent of interest, that is, the cancer patient is a responder. Thus, the marker for determining sensitivity to an anti-cancer agent can be used as a marker for actively continuing the therapy to a patient who is expected to receive a therapeutic effect. On the other hand, when p is less than 0.5, it can be determined that the cancer of the cancer patient is not sensitive to the anti-cancer agent of interest, that is, the cancer patient is a non-responder. When the cancer is not sensitive to the anti-cancer agent of interest, a beneficial effect of the anti-cancer agent is not expected. If the administration of the anti-cancer agent whose beneficial effect is not expected is carried out or continued, progress of cancer or aggravation of adverse effects would become a concern. Thus, the marker for determining sensitivity to an anti-cancer agent of the present invention can be used not only as a marker for actively continuing the therapy to a patient who is expected to receive a therapeutic effect, but also as a marker for avoiding progress of cancer or aggravation of adverse effects associated with continuous administration of the anti-cancer agent whose beneficial effect is not expected.

Among the formulas (1) to (3) described above which provide probability (p) of a cancer patient of interest being a responder, the formula (2) is preferred from the viewpoint of sensitivity.

To predict a total tumor diameter of a cancer patient of interest before starting the therapy by employing ASP and/or CSSG, the amount such as a concentration of ASP and/or CSSG in a biological sample from the cancer patient may be measured before administration of the anti-cancer agent, and then, a result of the measuring for each may be assigned to formula (4) or (5):

$$\text{Total tumor diameter (mm)} = 39.5 + 585.5 \times \text{ASP} \cdots (4)$$

(wherein, ASP represents a relative concentration (the relative concentration in the case of the concentration of internal standard solution for LC/MS being 1));

$$\text{Total tumor diameter (mm)} = 171.1 - 3540.6 \times \text{CSSG} \cdots (5)$$

(wherein, CSSG represents a relative concentration (the relative concentration in the case of the concentration of internal standard solution for LC/MS being 1)).

To predict prognosis in a therapy with an anti-cancer agent including oxaliplatin or a salt thereof, fluorouracil or a salt thereof, and levofolinate or a salt thereof, in particular, an anti-cancer agent including oxaliplatin or a salt thereof, fluorouracil or a salt thereof, levofolinate or a salt thereof, and bevacizumab, by employing one or more substances selected from the group consisting of 2-aminobutyric acid, CSSG, gamma-Glu-Cys, glycerol-3-phosphate, quinic acid, ASP, glycocholic acid, HYPX and lactic acid, the amount such as a concentration of one or more substances selected from the group consisting of 2-aminobutyric acid, CSSG, gamma-Glu-Cys, glycerol-3-phosphate, quinic acid, ASP, glycocholic acid, HYPX and lactic acid in a biological sample from a cancer patient may be measured before or after administration of the anti-cancer agent. Of these, it is preferred that the amount such as a concentration of one or more substances selected from the group consisting of ASP, CSSG and HYPX is measured. When the measured concentration of one or more substances selected from the group consisting of 2-aminobutyric acid, CSSG, gamma-Glu-Cys, glycerol-3-phosphate and quinic acid is evaluated as higher than a predetermined control level, it can be predicted that the prognosis is better than that in the case when the measured concentration is evaluated as lower than the predetermined control level. On the other hand, when the measured concentration of one or more substances selected from the group consisting of ASP, glycocholic acid, HYPX and lactic acid is evaluated as lower than a predetermined control level, it can be predicted that the prognosis is better than that in the case when the measured concentration is evaluated as higher than the predetermined control level. The prognosis prediction can be expressed by the length of progression-free survival (PFS), overall survival (OS), disease-free survival (DFS), or the like. Of these, PFS and/or OS is preferred, and OS is particularly preferred. Examples of the control level include a cut-off value. Examples of the cut-off for CSSG and HYPX include cut-off values of $2.223 \times 10^{-2}\leq$ and $\leq 1.050 \times 10^{-1}$, respectively, and these values are the same as the threshold values for responder. Other examples include a cut-off value of $0.2301\leq$ for 2-aminobutyric acid; a cut-off value of $<2.336 \times 10^{-2}$ for ASP; a cut-off value of $6.910 \times 10^{-3}\leq$ for gamma-Glu-Cys; a cut-off value of $5.207 \times 10^{-2}\leq$ for glycerol-3-phosphate; a cut-off value of $3.538 \times 10^{-2}\leq$ for glycocholic acid; a cut-off value of $<11.8839$ for lactic acid; and a cut-off value of $1.766 \times 10^{-2}\leq$ for quinic acid.

To carry out the method for determining sensitivity to an anti-cancer agent or a total tumor diameter of the present invention, it is preferred to use a kit comprising a protocol for measuring one or more substances selected from the group consisting of 2DG6P, 2MSE, CSSG, DOPM, GSSG, I4A, P2CB, 1-methyl-2-pyrrolidone, ASP, benzamide, glucaric acid, GL6P, Gly-Gly, HYPT and HYPX in a specimen. To carry out the method for predicting prognosis of the present invention, it is preferred to use a kit comprising a protocol for measuring one or more substances selected from the group consisting of 2-aminobutyric acid, CSSG, gamma-Glu-Cys, glycerol-3-phosphate, quinic acid, ASP, glycocholic acid, HYPX and lactic acid in a specimen. The kit comprises a reagent for measuring these metabolites, and a protocol (for example, a protocol which indicates a method for using the measuring reagent, a reference for determining whether or not the subject has sensitivity to the anti-cancer agent, or the like). Examples of the reference include standard concentrations for the metabolites, a concentration which is evaluated as a high level, a concentration which is evaluated as a low level, and factors or the degree of influence of the factors affecting the result of the measuring. These concentrations may be appropriately determined depending on an anti-cancer agent of interest. Using such a reference, the determination or prediction can be carried out as described above.

Moreover, by employing, as an index, expression variation of one or more substances selected from the group consisting of 2DG6P, 2MSE, CSSG, DOPM, GSSG, I4A, P2CB, 1-methyl-2-pyrrolidone, ASP, benzamide, glucaric acid, GL6P, Gly-Gly, HYPT, HYPX, 2-aminobutyric acid, gamma-Glu-Cys, glycerol-3-phosphate, quinic acid, glycocholic acid and lactic acid in a cancer cell line or a biological sample from a cancer-bearing animal in the presence of the anti-cancer agent, it becomes possible to select an anti-cancer agent sensitivity enhancer through screening.

Specifically, an anti-cancer agent sensitivity enhancer can be selected though screening by a method comprising adding or administering an anti-cancer agent and a test substance to a cancer cell line or a cancer-bearing animal and measuring a concentration of one or more substances selected from the group consisting of 2DG6P, 2MSE, CSSG, DOPM, GSSG, I4A, P2CB, 1-methyl-2-pyrrolidone, ASP, benzamide, glucaric acid, GL6P, Gly-Gly, HYPT, HYPX, 2-aminobutyric acid, gamma-Glu-Cys, glycerol-3-phosphate, quinic acid, glycocholic acid and lactic acid in the cancer cell line or a biological sample from the cancer-bearing animal; and selecting a test substance which enhances the sensitivity to the anti-cancer agent of the cancer cell line or the cancer-bearing animal based on a variation of the measured concentration. Herein, the expression variation includes the presence or absence of expression of the substances and/or increase or decrease (variation) of the expression amounts of the substances.

For example, an anti-cancer agent sensitivity enhancer can be selected through screening by employing, as an index, expression variation, specifically increase in concentration, of one or more substances selected from the group consisting of 2DG6P, 2MSE, CSSG, DOPM, GSSG, I4A, P2CB, 2-aminobutyric acid, gamma-Glu-Cys, glycerol-3-phosphate and quinic acid in the presence of the anti-cancer agent. That is, the substance which increases the concentration of these metabolites in vitro or in vivo enhances the sensitivity to the anti-cancer agent. For example, in vitro, a substance which increases the concentration of these metabolites in the presence of an anti-cancer agent in various cancer cell lines is a substance which enhances sensitivity to the anti-cancer agent (an anti-cancer agent sensitivity enhancer) in the cancer cell line. Moreover, in vivo, a substance which increases the concentration of these metabolites from before to after administration of an anti-cancer agent in a cancer-bearing animal is a substance which enhances sensitivity to the anti-cancer agent (an anti-cancer agent sensitivity enhancer) in the cancer-bearing animal.

Furthermore, for example, an anti-cancer agent sensitivity enhancer can be selected through screening by employing, as an index, expression variation, specifically decrease in concentration, of one or more substances selected from the group consisting of 1-methyl-2-pyrrolidone, ASP, benzamide, glucaric acid, GL6P, Gly-Gly, HYPT, HYPX, glycocholic acid and lactic acid in the presence of the anti-cancer agent. That is, the substance which decreases the concentration of these metabolites in vitro or in vivo enhances the sensitivity to the anti-cancer agent. For example, in vitro, a substance which decreases the concentration of these metabolites in the presence of an anti-cancer agent in various cancer cell lines is a substance which enhances sensitivity to the anti-cancer agent (an anti-cancer agent sensitivity enhancer). Moreover, in vivo, a substance which decreases the concentration of these metabolites from before to after administration of an anti-cancer agent in a cancer-bearing animal is a substance which enhances sensitivity to the anti-cancer agent (an anti-cancer agent sensitivity enhancer) in the cancer cell line.

By employing the thus-obtained anti-cancer agent sensitivity enhancer and the anti-cancer agent to be a target of sensitivity enhancement in combination, the therapeutic effect of the anti-cancer agent can be remarkably improved. The combination form of the anti-cancer agent sensitivity enhancer and the anti-cancer agent to be a target of sensitivity enhancement may be a single composition comprising both of them, and may be a combination of separate preparations comprising each. In addition, they may be administered through different routes. The anti-cancer agent to be a target of sensitivity enhancement is an anti-cancer agent including oxaliplatin or a salt thereof, fluorouracil or a salt thereof, and levofolinate or a salt thereof. This anti-cancer agent may be used in combination with an additional anti-cancer agent. Examples of the additional anti-cancer agent include, but are not particularly limited to, cyclophosphamide, ifosfamide, thiotepa, melphalan, busulfan, nimustine, ranimustine, dacarbazine, procarbazine, temozolomide, cisplatin, carboplatin, nedaplatin, methotrexate, pemetrexed, tegaful/uracil, doxifluridine, tegaful/gimeracil/oteracil, capecitabine, cytarabine, enocitabine, gemcitabine, 6-mercaptopurine, fuludarabin, pentostatin, cladribine, hydroxyurea, doxorubicin, epirubicin, daunorubicin, idarubicine, pirarubicin, mitoxantrone, amurubicin, actinomycin D, bleomycine, pepleomycin, mytomycin C, aclarubicin, zinostatin, vincristine, vindesine, vinblastine, vinorelbine, paclitaxel, docetaxel, irinotecan, SN-38, nogitecan, topotecan, etoposide, prednisolone, dexamethasone, tamoxifen, toremifene, medroxyprogesterone, anastrozole, exemestane, letrozole, rituximab, imatinib, gefitinib, gemtuzumab/ozogamicin, bortezomib, erlotinib, cetuximab, bevacizumab, sunitinib, sorafenib, dasatinib, panitumumab, asparaginase, tretinoin, arsenic trioxide, or a salt thereof or an active metabolite thereof. Among them, irinotecan, SN-38 or a salt thereof or bevacizumab is preferred, and bevacizumab is particularly preferred.

Examples

Next, the present invention will be described in more detail by way of Examples, which should not be construed as limiting the invention thereto.

(1) Method

(a) Reagents

For dissolution and sample preparation, methanol for LC/MS (manufactured by Wako Pure Chemical Industries, Ltd.), chloroform for HPLC (manufactured by Wako Pure Chemical Industries, Ltd.) and reverse osmosis water (Direct-Q UV, manufactured by Millipore) were used.

As the internal standard solutions for LC/MS (cation), Internal Standard Solution Compound C1 (ISC1) and Internal Standard Solution Compound C2 (ISC2) were used. The ISC1 is an aqueous solution containing 10 mM L-methionine sulfone (manufactured by Human Metabolome Technologies Inc.), and the ISC2 is an aqueous solution containing 10 mM L-Arginine-$^{13}C_6$ hydrochloride (manufactured by Sigma-Aldrich Co. LLC.), L-Asparagine-$^{15}N_2$ monohydrate (manufactured by Cambridge Isotope Laboratories, Inc.), β-Alanine-$^{13}C_3$, $^{15}N$ (manufactured by Sigma-Aldrich Co. LLC.) and Tubercidin (manufactured by Sigma-Aldrich Co. LLC.).

As the internal standard solutions for LC/MS (anion), Internal Standard Solution Compound A1 (ISA1) and Internal Standard Solution compound A2 (ISA2) were used. The ISA1 is an aqueous solution containing 10 mM D-camphor-10-sulfonic acid sodium salt (manufactured by Human Metabolome Technologies), and the ISA2 is an aqueous solution containing 10 mM chloranilic acid (manufactured by Tokyo Chemical Industry Co., Ltd.).

These internal standard solutions were used to normalize the signal intensity and to adjust the migration time. Moreover, the ISC1 and ISA1 were also used to calculate the relative concentration of each of the obtained metabolites.

(b) Clinical samples

(b-1) Patient backgrounds

Among histologically confirmed advanced colorectal cancer (ACRC) patients, the total of 68 patients who were eligible as subjects for the primary therapy of standard chemotherapy were selected, and from the eligible patients, serum samples were prospectively collected. Registration criteria of patients in this study (eligibility) were as follows.

- The age at the time of registration is 20 years old or more,
- Performance status (PS) of Eastern Corporative Oncology Group (ECOG) is 0 or 1,
- The cancer of the patient has been histopathologically confirmed as colorectal cancer,
- The cancer is an advanced or recurrent disease which is not resectable and has not been treated chemotherapeutically (only in the case where the cancer is treated by an adjuvant post-operative chemotherapy with a 5-FU-based drug, registration is possible if the adjuvant post-operative chemotherapy had been completed by 6 months before the confirmed recurrence date),
- The predicted survival of the patient is 3 months or more,
- There is no serious dysfunction in the patient's major organs
- Before the registration of this test, a written consent for participation of this test including a genetic polymorphism testing and a proteome metabolome analysis, which is signed by the patient himself/herself and dated, has been submitted.

(b-2) Therapy for patient

All patients received, as primary chemotherapy, 5 mg/kg of bevacizumab (BV) intravenously over 30 to 90 minutes, and subsequently, 85 mg/m$^2$ of oxaliplatin (L-OHP) and 200 mg/m$^2$ of levofolinate (l-LV) intravenously over 120 minutes. Then, they received 400 mg/m$^2$ of 5-FU via bolus intravenous injection, and subsequently, 2400 mg/m$^2$ of 5-FU via continuous intravenous infusion over 46 hours (mFOLFOX6 therapy). This therapy was repeatedly performed once every two weeks.

Even after the L-OHP administration had been suspended, combined administration of 1-LV and 5-FU (sLV5FU2) was continued with or without of BV treatment as needed. This reduced combined administration of l-LV and 5-FU (sLV5FU2) was also considered as a test therapy.

The test therapies were continued up to 24 cycles unless there was no hindrance such as progress of disease, development of adverse event which requires suspension of further test therapy, decision of doctor, refusal from patient on continuation of test therapy, or transition to curative surgical resection of a tumor.

(b-3) Evaluation of anti-tumor effect

The anti-tumor effect was evaluated based on the Response Evaluation Criteria in Solid Tumors Guideline 1.0 (RECIST) by an independent external review board.

The total tumor diameter before the therapy was measured with the images taken within one month before registration by computer tomographic equipment or magnetic resonance imaging. The total tumor diameter after starting the therapy was measured with the images taken repeatedly once in 8 weeks, by the same method as that before the therapy.

(b-4) Collection of sample

Blood samples were collected within two weeks before starting the chemotherapy. Blood samples were also collected, during the period after starting the chemotherapy until the suspension of oxaliplatin therapy, two weeks after performing the chemotherapy in each therapy cycle.

The collected blood specimen was coagulated by leaving it for 15 minutes at room temperature, and then centrifuged at 4°C for 30 minutes under 3,000 rpm. The obtained serum was then aliquoted into four polypropylene tubes at an equal amount, and quickly frozen with liquid nitrogen. All of these procedures were completed within 1 hour from the blood collection. The serum samples were stored at -80°C until analysis.

(b-5) Preparation of sample

Samples were prepared in accordance with a previously reported method (J Proteome Res. 2003 Sep-Oct; 2(5):488-94, Metabolomics. 2010 Mar; 6(1):78-95, Metabolomics. 2013 Apr;9(2): 444-453). The serum sample was thawed on ice, and 200 $\mu$L of the obtained serum was put into a centrifuge tube containing 1800 $\mu$L of methanol together with internal standard (10 $\mu$M of ISA1 or ISC1), 2000 $\mu$L of chloroform and 800 $\mu$L of reverse osmosis water, and they were mixed. After vortexing, the mixture was centrifuged at 4°C under 4600 g for 5 minutes. Then, 1500 $\mu$L of the resultant upper layer was displaced for protein removal in 5 kDa filter (manufactured by Millipore) and centrifugally filtered at 4°C under 9100 g for 2 to 4 hours. The filtered solution was dried with vacuum centrifuge. The dried filtered solution was dissolved on ice in 50 $\mu$L of reverse osmosis water containing ISC2 or ISA2 at the final concentration of 0.1 mM, and then the solution was put into an analysis vial, and centrifuged at 4°C under 1000 g for 10 minutes. The resultant was soon analyzed by CE-TOF MS.

(c) Measurement of metabolites in sample by CE-TOF MS

All samples were measured in duplicate. Using CE-Q-TOF MS under a cationic measurement condition and CE-TOF MS under an anionic measurement condition, metabolites with a mass number of 1000 or less were comprehensively measured.

(c-1) Cationic metabolite measurement condition

1) Measurement equipment

For measuring cationic metabolites, Agilent 6530 Accurate-Mass Q-TOF MS system (manufactured by Agilent Technologies, Inc.) equipped with Agilent 7100 CE system was used. As the capillary, a fused silica capillary (inner diameter 50 $\mu$m, total length 80 cm) of catalogue number (Cat. No.) H3305-2002 available from Human Metabolome Technologies, Inc. (HMT) was used. The buffer used was a buffer of Cat. No.3301-1001 available from HMT. The measuring was performed with applied voltage of +27 kv and at capillary temperature of 20°C. The sample injection was carried out at 50 mbar over 10 seconds by the pressure method.

2) Analysis condition of Time-of-Flight Mass Spectrometer (Q-TOF MS)

In the analysis, cation mode was used. The following condition was set: ionizing voltage of 4 kv, fragmentor voltage of 80 v, skimmer voltage of 50 v, and octRFV voltage of 650 v. Nitrogen was used as the drying gas. The temperature was set to 300°C, and the pressure was set to 5 psig. The sheath liquid used was a sheath liquid of Cat. No. H3301-1020 available from HMT. The reference mass was set at m/z 65.059706 and m/z 622.08963.

(c-2) Anionic metabolite measurement condition

1) Measurement equipment

For measuring anionic metabolites, Agilent 6210 TOF system (manufactured by Agilent Technologies, Inc.) equipped with Agilent 1600 CE system was used. The same capillary and the same temperature condition as those for anion was used. The buffer used was a buffer of Cat. No. H3302-1021 available from HMT. The applied voltage was set at 30 kv. The sample injection was carried out at 50 mbar over 25 seconds by the pressure method.

2) Analysis condition of Time-of-Flight Mass Spectrometer (TOF MS)

In the analysis, new anion mode was used. The following condition was set: ionizing voltage of 3.5 kv, fragmentor voltage of 125 v, skimmer voltage of 50 v, and octRFV voltage of 175 v. The same drying gas and the same Sheath liquid were used in the same condition as those set for cation. The reference mass was set at m/z 51.013854 and m/z 680.035541.

(c-3) Data processing

To obtain peak information including m/z, migration time (MT) and peak area, raw data of the peaks observed by CE-Q-TOF MS or CE-TOF MS were processed by Master Hands automated integration software version 2.0 (manufactured by Keio University). With this software, all peaks were found, noise was removed, and data matrix including annotation and relative peak area of metabolites was generated. The peaks were annotated by the names of metabolites which were assumed from Metabolites Database of HMT, based on m/z from CE and MT from TOF MS. The conditions of MT, m/z, and minimum S/N ratio for annotating anion peaks were 1.5 minutes, 50ppm, and 20, respectively, and the conditions of those for annotating cation peaks were 0.5 minutes, 50 ppm, and 20, respectively.

The relative concentration of each of the annotated metabolites was calculated by dividing the peak area of each metabolite by area of ISC1 (cation) or ISA1 (anion).

In the CE-Q-TOF MS and CE-TOF MS analysis, the anti-tumor effect for individual patients was masked against analyzers.

A list of the processed peaks was outputted for further statistical analysis. For the statistical analysis, the average of relative concentration of each of the annotated metabolites measured in duplicate was taken.

(d) Statistical analysis

(d-1) Analysis method 1

For clinical and metabolomics data processing and statistical analysis, JMP 12, 64 bit version (manufactured by SAS Institute Inc.) was used with Microsoft Windows 7.

In this test, 68 serum samples before starting the test therapy were obtained from the 68 patients. To study factors for predicting therapeutic effects, data of the metabolites which were obtained from the 68 serum samples before starting the test therapy was used.

In this test, the maximal anti-tumor effect during the test therapy period was grasped, and a group of patient having therapeutic response (responder) and a group of patient not having therapeutic response (non-responder) were defined in accordance with the following.

- responder (R): a patient whose maximum effect during the test therapy period shows complete response or partial response as a result of the diagnostic imaging by radiation diagnostician in accordance with RECIST criteria
- non-responder (N-R): a patient whose maximum effect during the test therapy period shows stable disease or progressive disease as a result of the diagnostic imaging by radiation diagnostician in accordance with RECIST criteria.

To verify the difference in the patient backgrounds, $\chi$-square test was used. To study the difference between the N-R group and the R group of each metabolite, t-test (Welch) was used. The substances which became significant as a result of these tests were employed as candidate substances for the marker for determining sensitivity to an anti-cancer agent. To confirm association between the candidate substances to each other and relation of each candidate substance to the patient background, Pearson correlation coefficient was used. To establish an anti-cancer agent sensitivity determination model, nominal logistic regression analysis of univariate and multivariate with variable increase and decrease technique was performed. To evaluate a predictability of each candidate substance, receiver operating characteristic (ROC) was used. In the logistic regression analysis of multivariate, p value was calculated in which multiplicity was adjusted by the false discovery rate finding method of Benjamini and Hochberg (BH-FDR) to control false discovery rate (Journal of the Royal Statistical Society, Series B, 57, 289-300). The survival curve and therapy period were estimated by Kaplan-Meier method, and the difference in the curve was analyzed by Log-rank test. The evaluation of prediction prognosis of metabolites which were the candidate substances was examined by univariate and multivariate analysis using the Cox proportional hazard model.

In addition, the metabolites which became significant in the hazard analysis were divided into two groups based on the threshold for responder or the like, and then Kaplan-Meier curves were depicted for each of the groups, and analysis was performed by Log-rank test.

In any of these analysis, p value <0.05 was considered as statistically significant.

(d-2) Analysis method 2

For clinic and metabolomics data processing and statistical analysis, JMP 12, 64 bit version (manufactured by SAS Institute Inc.) was used with Microsoft Windows 7.

In this test, 68 serum samples before starting the test therapy were obtained from the 68 patients. To study factors for predicting therapeutic effects, data of the metabolites which were obtained from the 68 serum samples before starting the test therapy was used.

The responder (R) and non-responder (N-R) were defined in the same way as in (d-1).

To study the differences between the N-R group and the R group for each metabolite, nominal logistic analysis was comprehensively performed for the metabolites, and the metabolites which became significant in whole of the model were employed as candidate substances for the marker for determining sensitivity to an anti-cancer agent. The ROC curves and their AUC for these metabolites, and the sensitivity, specificity and accuracy of each metabolite were determined in the same way as in (d-1).

Next, to establish an effect predictive model based on the candidate substances for the marker for determining sensitivity to an anti-cancer agent, cut-off values for each of the metabolites which became significant in univariate analysis were obtained from the ROC curves. Further, based on the obtained cut-off values, binarization was performed. Using the binarized values, variables were selected with variable increase technique, by employing the Bayesian information criterion (BIC) as an index in the STEP WISE method. Then, with the selected variables, multivariate nominal logistic regression analysis was performed. To evaluate the effect predictability of the candidate substances for the marker for determining sensitivity to an anti-cancer agent, receiver operating characteristics (ROC) was used.

To evaluate the actual clinical effects and the effect predictive performance based on the prediction model, a confusion matrix was generated, and the predictive performance for responder was evaluated in terms of sensitivity, specificity, and accuracy.

In order to study the prognosis predictive performance of the effect prediction model, overall survival was estimated by the Kaplan-Meier method, and the difference between the responder and non-responder was studied by Log-rank test.

Furthermore, the blood concentration before starting the therapy and overall survival of each patient were used to analyze prognosis predictive metabolites by COX proportional hazard model. In these statistical analysis, p value <0.05 was considered as significant. In addition, the study was conducted for metabolites which were detected in 75% or more of the tested patients.

(2) Results

(2-1) Results in analysis method 1

(a) Substances which showed significant difference between R group and N-R group

As shown in Table 1, 29 patients were defined as N-R, and 39 patients were defined as R. Difference in patient backgrounds was not observed between the R group and the N-R group.

[Table 1]

|  | | N-R | R | *p* value |
|---|---|---|---|---|
| Sex | | | | |
| | Male | 17 | 21 | 0.8063 |
| | Female | 12 | 18 | |
| Age | | | | |
| | Media(years) | 64 | 63 | 0.1106 |
| | Range | 54-78 | 28-81 | |
| ECOG PS | | | | |
| | 0 | 25 | 29 | 0.3638 |
| | 1 | 4 | 10 | |
| Primary site | | | | |
| | Colon | 19 | 25 | 1.0000 |
| | Rectum | 10 | 14 | |
| Primary site | | | | |
| | Resected | 21 | 33 | 0.2413 |
| | Exist | 8 | 6 | |

(continued)

| Baseline total tumor length (mm) | | | |
|---|---|---|---|
| Media | 90.7 | 97.9 | 0.6204 |
| Range | 16.4-261.0 | 12.2-247.3 | |
| Histological type | | | |
| Well-differentiated | 8 | 8 | 0.533 |
| Moderately differentiated | 17 | 26 | (Well-differentiated |
| Poorly differentiated | 2 | 4 | vs. |
| Mucinous | 2 | 0 | Moderately differentiated |
| Mixture of well-differenciated and moderately differentiated | 0 | 1 | vs. others) |
| Number of metastatic organs | | | |
| 1 | 12 | 18 | 0.8063 |
| ≥2 | 17 | 21 | |
| Adjuvant chemotherapy | | | |
| Yes | 28 | 36 | 0.6306 |
| No | 1 | 3 | |

N-R, Non Responder. R, Responder
ECOG PS, Eastern Cooperative Oncology Group Performance status.

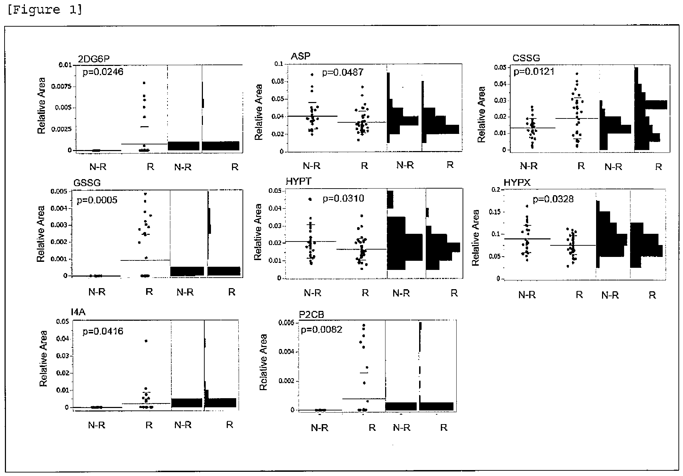

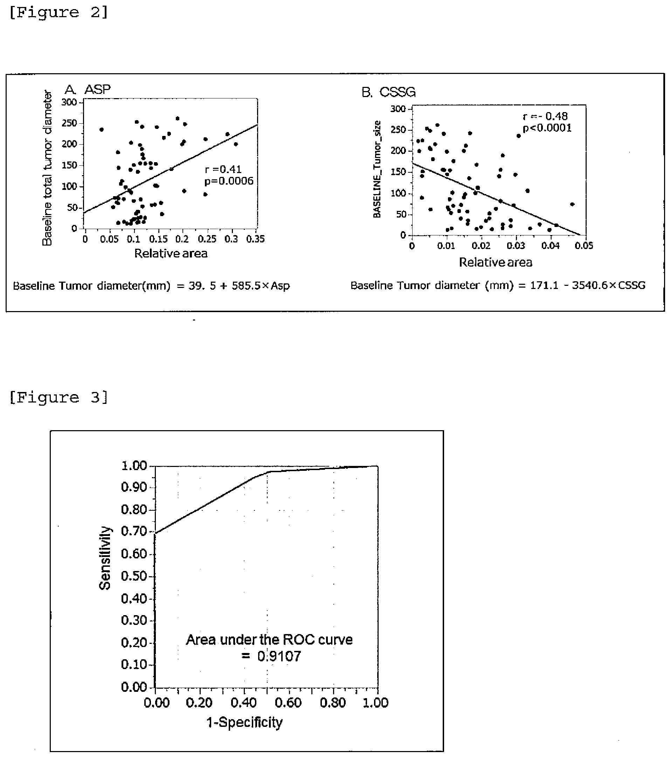

A comprehensive t-test (Welch) was carried out for 380 metabolites whose expressions were observed in the serum samples obtained before starting the test therapy. As a result, as shown in Figure 1, 2-deoxyglucose 6-phosphate (2DG6P), cysteine-glutathione disulphide (CSSG), oxidized glutathione (GSSG), imidazole-4-acetate (I4A) and pyridine-2-carboxylic acid butyl ester (P2CB) were found as metabolites which showed high level with statistically significance in the R group compared to those in the N-R group. The p values for each were 0.0246, 0.0121, 0.0005, 0.0416, and 0.0082, respectively. Furthermore, the metabolites which showed low level with statistically significance in the R group compared to those in the N-R group were aspartate (ASP), hypotaurine (HYPT) and hypoxanthine (HYPX). The p values for each were 0.0487, 0.0310, and 0.0328, respectively. In particular, 2DG6P, GSSG, I4A and P2CB were only found in the R group patients.

(b) Correlation of total tumor diameter before starting test therapy and each substance

As shown in Figure 2, ASP and CSSG showed significant correlation with a total tumor diameter before starting the test therapy. The regression formula, correlation coefficient (r) and p value for each were as follows:

Regression formula:

ASP: 39.5 + 585.5 × ASP,
CSSG: 171.1 - 3540.6 × CSSG;

Correlation coefficient and p value:

ASP: r = 0.41 (p = 0.0006),
CSSG : r = -0.48 (p < 0.0001).

(c) Calculation of anti-cancer agent sensitivity determination model

For eight candidate substances for the marker for determining sensitivity to an anti-cancer agent shown in (a), univariate nominal logistic analysis was performed with an objective variable of whether a responder or non-responder. From this analysis, the cut-off value, probability value, and area under the curve ROC for each substance were obtained. Each result is shown in Table 2. The cut-off values for responders in each substance were as follows. 2DG6P ($5.304 \times 10^{-4} \leq$) ; ASP ($\leq 3.401 \times 10^{-2}$) ; CSSG ($2.223 \times 10^{-2} \leq$) ; GSSG ($1.061 \times 10^{-3} \leq$); HYPT ($\leq 1.837 \times 10^{-2}$) ; HYPX

($\leq 1.050 \times 10^{-1}$) ; I4A ($3.316 \times 10^{-3}\leq$) ; and P2CB ($5.952 \times 10^{-4}\leq$). Most of the candidate substances have p value of less than 0.01. ASP and CSSG showed particularly good AUC (0.7<). Furthermore, specificity and true positive predictive value of 2DG6P, GSSG, I4A and P2CB were both 1.0000 for each substance alone. CSSG showed the smallest p value and the maximum AUC. HYPX showed the highest in sensitivity and negative predictive value.

[Table 2]

| Candidate TRPs | Responder cut off values | Univariate analysis | | Sensitivity | Specificity | Positive predictive value | Negative predictive value |
|---|---|---|---|---|---|---|---|
| | | p value | AUC of ROC | | | | |
| 2DG6P | $5.304 \times 10^{-4}\leq$ | 0.0077 | 0.5769 | 0.1538 | 1.0000 | 1.0000 | 0.4677 |
| ASP | $\leq 3.401 \times 10^{-2}$ | 0.0006 | 0.7082 | 0.6923 | 0.7241 | 0.7714 | 0.6364 |
| CSSG | $2.223 \times 10^{-2}\leq$ | <.0001 | 0.7091 | 0.4872 | 0.9310 | 0.9047 | 0.5745 |
| GSSG | $1.061 \times 10^{-3}\leq$ | <.0001 | 0.6539 | 0.3077 | 1.0000 | 1.0000 | 0.5179 |
| HYPT | $\leq 1.837 \times 10^{-2}$ | 0.0243 | 0.6348 | 0.7179 | 0.5517 | 0.6829 | 0.5925 |
| HYPX | $\leq 1.050 \times 10^{-1}$ | 0.0014 | 0.6468 | 0.9487 | 0.3448 | 0.6607 | 0.8333 |
| I4A | $3.316 \times 10^{-3}\leq$ | 0.0009 | 0.6154 | 0.2308 | 1.0000 | 1.0000 | 0.4915 |
| P2CB | $5.952 \times 10^{-4}\leq$ | 0.0019 | 0.6026 | 0.2051 | 1.0000 | 1.0000 | 0.4833 |

\* Candidate TRPs: Candidates for a marker for determining sensitivity to an anti-cancer agent

The anti-cancer agent sensitivity determination model was calculated from multivariate nominal logistic model with variable increase and decrease technique. As a result, 2DG6P, CSSG, HYPT, I4A and P2CB were left, and as shown in Table 3, FDR p values for each were 0.00082, 0.00996, 0.00222, 0.01163 and 0.01163, respectively.

[Table 3]

| Predictive markers | Coefficient | Standard error | FDR p value | Lower 95% CI | Upper 95% CI |
|---|---|---|---|---|---|
| (Intercept) | 3.067 | 1.086 | | 1.381 | 6.036 |
| 2DG6P | $-1.997 \times 10$ | $3.176 \times 10^{3}$ | 0.00082 | $-6.245 \times 10^{3}$ | $6.205 \times 10^{3}$ |
| CSSG | -2.844 | 1.253 | 0.00996 | -6.016 | $-7.381 \times 10^{-1}$ |
| HYPT | -2.864 | 1.124 | 0.00222 | -5.860 | -1.034 |
| I4A | $-1.768 \times 10$ | $2.726 \times 10^{3}$ | 0.01163 | $-5.361 \times 10^{3}$ | $5.326 \times 10^{3}$ |
| P2CB | $-1.781 \times 10$ | $2.918 \times 10^{3}$ | 0.01163 | $-5.738 \times 10^{3}$ | $5.702 \times 10^{3}$ |

The obtained anti-cancer agent sensitivity determination model was as shown in the following formula (1) :

$$p = \frac{1}{1+e^{-(-3.067+19.971\times(2DG6P)+2.844\times(CSSG)+2.864\times(HYPT)+17.68\times(I4A)+17.81\times(P2CB))}} \qquad (1)$$

(wherein, 2DG6P, CSSG, I4A and P2CB represent 1, respectively, when a result of the measuring for each substance is equal to or more than a respective cut-off value, and represent 0, respectively, when the result of the measuring for each is less than the respective cut-off value; and HYPT represents 1 when a result of the measuring for HYPT is equal to or less than a cut-off value, and represents 0 when the result of the measuring for HYPT is more than the cut-off value).

The model represents a formula to determine whether a patient is a responder or not. The p value represents probability of a patient being a responder, and when the p value is 0.5 or more, the patient is determined as a responder.

In this anti-cancer agent sensitivity determination model, AUC of the ROC curve was 0.9107, and the cut-off value was -2.640 or less (Figure 3). The sensitivity, specificity, true positive predictive value and false positive predictive value thereof were 0.6923, 1.0000, 1.0000, and 0.7073, respectively.

(d) Analysis of PFS and OS by concentration of CSSG

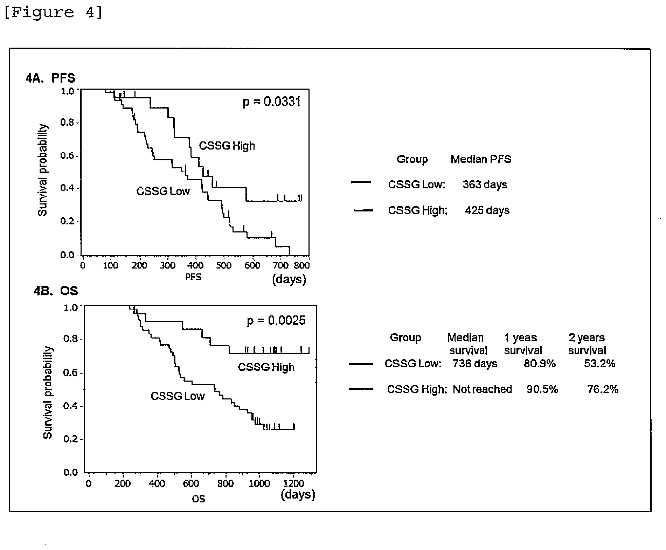

The analysis of progression-free survival (PFS) and overall survival (OS) was performed according to the concentration of CSSG (the concentration equal to and more than the cut-off value described in the above (c) was considered CSSG High, and the concentration less than the cut-off value was considered CSSG Low). As a result, CSSG High group showed significantly longer PFS (p = 0.0331) and OS (p = 0.0025) than CSSG Low group (Figure 4A and 4B).

Median PFS of CSSG High group was 425 days, and that of CSSG Low group was 363 days. One-year and 2-years survival rates of CSSG High group were 90.5% and 76.2% respectively, while 1-year and 2-years survival rates of CSSG Low group were 80.9% and 53.2% respectively.

(2-2) Results in analysis method 2

(a) Substances which showed differences in logistic analysis between R group and N-R group

As shown in Table 1, there was no difference in patient backgrounds between the R group and the N-R group.

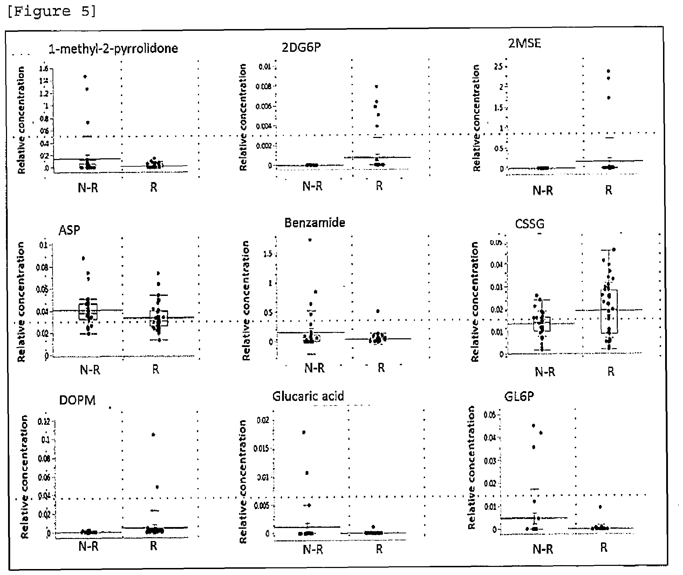

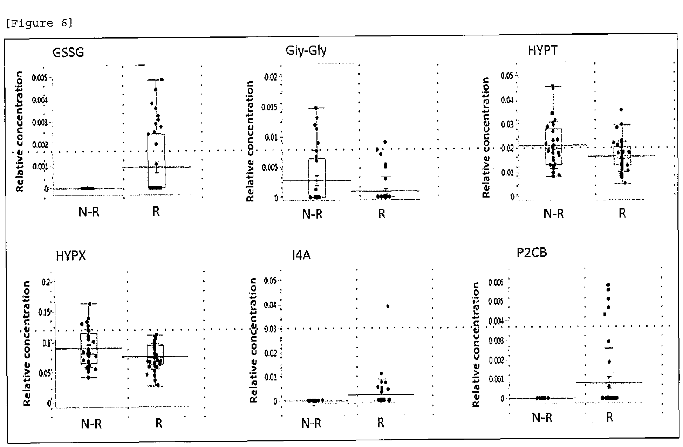

For 480 metabolites whose expression was observed in the serum samples obtained before the test therapy, nominal logistic analysis was comprehensively performed with interest variable of therapeutic response (whether a responder or non-responder). As a result, 15 metabolites shown in Table 4 became significant. Distributions of each metabolite in the responder group and non-responder group are shown in Figures 5 and 6.

[Table 4]

| Metabolites | p value (Whole Model) |
| --- | --- |
| 2DG6P | 0.0077 |
| 2MSE | 0.0025 |
| CSSG | 0.0179 |
| DOPM | 0.0031 |
| GSSG | <0.0001 |
| I4A | 0.0009 |
| P2CB | 0.0019 |
| 1-methyl-2-pyrrolidone | 0.0365 |
| ASP | 0.0383 |
| Benzamide | 0.0267 |
| Glucaric acid | 0.0328 |
| GL6P | 0.0167 |
| Gly-Gly | 0.0379 |
| HYPT | 0.0188 |
| HYPX | 0.022 |

Furthermore, binarization was performed based on the cut-off value obtained from the ROC curve, and p value, sensitivity, accuracy, responder predictive value, and non-responder predictive for binarization were calculated. The results each are shown in Table 5. The cut-off values in each substance were as follows: 1-methyl-2-pyrrolidone ($\leq 8.422 \times 10^{-2}$) ; 2DG6P ($5.304 \times 10^{-4} \leq$); 2MSE ($1.404 \times 10^{-3} \leq$) ; ASP ($\leq 3.401 \times 10^{-2}$) ; benzamide ($\leq 9.859 \times 10^{-2}$); CSSG ($2.223 \times 10^{-2} \leq$) ; DOPM ($1.153 \times 10^{-3} \leq$) ; glucaric acid ($\leq 1.058 \times 10^{-3}$) ; GL6P ($\leq 8.167 \times 10^{-4}$) ; GSSG ($1.061 \times 10^{-3} \leq$) ; Gly-Gly ($\leq 5.349 \times 10^{-3}$) ; HYPT ($\leq 1.837 \times 10^{-2}$) ; and HYPX ($\leq 1.050 \times 10^{-1}$) ; I4A ($3.316 \times 10^{-3} \leq$) ; P2CB ($5.952 \times 10^{-4} \leq$). Of these, ASP and CSSG showed particularly good AUC ($0.7 \leq$). Furthermore, specificity and true positive predictive values of 2DG6P, GSSG, I4A and P2CB were both 1.0000 for each substance alone.

[Table 5]

| Candidate TRPs | Responder cut off values | Univariate analysis | | Sensitivity | Specificity | Positive predictive value | Negative predictive value |
|---|---|---|---|---|---|---|---|
| | | p value | AUC of ROC | | | | |
| 2DG6P | $5.304 \times 10^{-4} \leq$ | 0.0077 | 0.58 | 0.154 | 1.000 | 1.000 | 0.515 |
| 2MSE | $1.404 \times 10^{-3} \leq$ | 0.006 | 0.63 | 0.333 | 0.931 | 0.867 | 0.588 |
| CSSG | $2.223 \times 10^{-2} \leq$ | <0.0001 | 0.71 | 0.487 | 0.931 | 0.905 | 0.676 |
| DOPM | $1.153 \times 10^{-3} \leq$ | 0.0019 | 0.67 | 0.436 | 0.897 | 0.850 | 0.632 |
| GSSG | $1.061 \times 10^{-3} \leq$ | <0.0001 | 0.65 | 0.308 | 1.000 | 1.000 | 0.603 |
| I4A | $3.316 \times 10^{-3} \leq$ | 0.0009 | 0.62 | 0.231 | 1.000 | 1.000 | 0.559 |
| P2CB | $5.952 \times 10^{-4} \leq$ | 0.0019 | 0.60 | 0.205 | 1.000 | 1.000 | 0.544 |
| 1-methyl-2-pyrrolidone | $\leq 8.422 \times 10^{-2}$ | 0.0479 | 0.58 | 0.949 | 0.207 | 0.617 | 0.632 |
| ASP | $\leq 3.401 \times 10^{-2}$ | 0.0012 | 0.70 | 0.667 | 0.724 | 0.765 | 0.691 |
| Benzamide | $\leq 9.859 \times 10^{-2}$ | 0.0272 | 0.60 | 0.923 | 0.276 | 0.632 | 0.647 |
| Glucaric acid | $\leq 1.058 \times 10^{-3}$ | 0.0213 | 0.55 | 1.000 | 0.103 | 0.600 | 0.618 |
| GL6P | $\leq 8.167 \times 10^{-4}$ | 0.1039 | 0.56 | 0.949 | 0.172 | 0.607 | 0.618 |
| Gly-Gly | $\leq 5.349 \times 10^{-3}$ | 0.0272 | 0.60 | 0.923 | 0.276 | 0.632 | 0.647 |
| HYPT | $\leq 1.837 \times 10^{-2}$ | 0.0428 | 0.62 | 0.692 | 0.552 | 0.675 | 0.632 |
| HYPX | $\leq 1.050 \times 10^{-1}$ | 0.0051 | 0.63 | 0.923 | 0.345 | 0.655 | 0.676 |

(B) Calculation of anti-cancer agent sensitivity determination model

The anti-cancer agent sensitivity determination model was calculated from multivariate nominal logistic model with variable increase and decrease technique. As a result, as shown in Table 6, a seven metabolites model was calculated. In this model, 2DG6P, 2MSE, ASP, CSSG, DOPM, GL6P and HYPT were selected. FDR p values for each were 0.02161, 0.02851, 0.00679, 0.00023, 0.00023, 0.03439 and 0.00023, respectively. Further, a three metabolites model was calculated from the metabolites which became also significant in parameters in univariate analysis. In this three metabolites model, CGGS, DOPM and HYPT were selected. FDR p values for each were 0.00001, 0.00032 and 0.00281, respectively.

[Table 6]

| | Predictive markers | Coefficient | Standard error | FDR $p$ value | Lower 95% CI | Upper 95%CI |
|---|---|---|---|---|---|---|
| 7 metabolites model | (Intercept) | −11.5959 | $1.357 \times 10^3$ | | $-2.671 \times 10^3$ | $2.649 \times 10^3$ |
| | 2DG6P | 10.2190 | $1.357 \times 10^3$ | 0.02161 | $5.248 \times 10^{-1}$ | $2.670 \times 10^3$ |
| | 2MSE | 1.4778 | $7.473 \times 10^{-1}$ | 0.02851 | $1.794 \times 10^{-1}$ | 3.314 |
| | ASP | −1.4976 | $5.796 \times 10^{-1}$ | 0.00679 | −2.794 | $-4.556 \times 10^{-1}$ |
| | CSSG | 2.0937 | $7.311 \times 10^{-1}$ | 0.00023 | $9.129 \times 10^{-1}$ | 3.992 |
| | DOPM | 2.2258 | $7.818 \times 10^{-1}$ | 0.00023 | $9.683 \times 10^{-1}$ | 4.184 |
| | GL6P | −1.6623 | $9.230 \times 10^{-1}$ | 0.03439 | −3.841 | $-1.098 \times 10^{-1}$ |
| | HYPT | −2.3200 | $8.452 \times 10^{-1}$ | 0.00023 | −4.416 | $-9.719 \times 10^{-1}$ |
| 3 metabolites model | (Intercept) | −1.7898 | $5.568 \times 10^{-1}$ | | −3.059 | $-8.265 \times 10^{-1}$ |
| | CSSG | 1.8701 | $5.242 \times 10^{-1}$ | 0.00001 | $9.770 \times 10^{-1}$ | 3.085 |
| | DOPM | 1.4081 | $4.572 \times 10^{-1}$ | 0.00032 | $6.096 \times 10^{-1}$ | 2.464 |
| | HYPT | −1.0869 | $4.229 \times 10^{-1}$ | 0.00281 | −2.077 | $-3.458 \times 10^{-1}$ |

The anti-cancer agent sensitivity determination models (seven metabolites model and three metabolites model) are as shown by the following formulas (2) and (3), respectively.

7 metabolites model

$$p = \frac{1}{1+e^{(-11.5959-((2DG6P)+(2MSE)+(ASP)+(CSSG)+(DOPM)+(GL6P)+(HYPT)))}} \qquad (2)$$

(wherein, 2DG6P represents 10.2190 when a result of the measuring for 2DG6P is equal to or more than a cut-off value, and represents -10.2190 when the result of the measuring is less than the cut-off value; 2MSE represents 1.4778 when a result of the measuring for 2MSE is equal to or more than a cut-off value, and represents -1.4778 when the result of the measuring is less than the cut-off value; ASP represents -1.4976 when a result of the measuring for ASP is equal to or more than a cut-off value, and represents 1.4976 when the result of the measuring is less than the cut-off value; CSSG represents 2.0937 when a result of the measuring for CSSG is equal to or more than a cut-off value, and represents -2.0937 when the result of the measuring is less than the cut-off value; DOPM represents 2.2258 when a result of the measuring for DOPM is equal to or more than a cut-off value, and represents -2.2258 when the result of the measuring is less than the cut-off value; GL6P represents -1.6623 when a result of the measuring for GL6P is equal to or more than a cut-off value, and represents 1.6623 when the result is less than the cut-off value; and HYPT represents -2.3200 when a result of the measuring for HYPT is equal to or more than a cut-off value, and represents 2.3200 when the result of the measuring is less than the cut-off value, and wherein the cut-off for 2DG6P is $5.304 \times 10^{-4}$; the cut-off for 2MSE is $1.404 \times 10^{-3}$; the cut-off for ASP is $3.401 \times 10^{-2}$; the cut-off for CSSG is $2.223 \times 10^{-2}$; the cut-off for DOPM is $1.153 \times 10^{-3}$; the cut-off for GL6P is $8.167 \times 10^{-4}$; and the cut-off for HYPT is $1.837 \times 10^{-2}$).

3 metabolites model

$$p = \frac{1}{1+e^{(-1.7898-((CSSG)+(DOPM)+(HYPT)))}} \qquad (3)$$

(wherein, CSSG represents 1.8701 when a result of the measuring for CSSG is equal to or more than a cut-off value, and represents -1.8701 when the result of the measuring is less than the cut-off value; DOPM represents 1.4081 when a result of the measuring for DOPM is equal to or more than a cut-off value, and represents -1.4081 when the result of the measuring is less than the cut-off value; and HYPT represents -1.0869 when a result of the measuring for HYPT is equal to or more than a cut-off value, and represents 1.0869 when the result of the measuring is less than the cut-off value, and wherein, the cut-off for CSSG is $2.223 \times 10^{-2}$; the cut-off for DOPM is $1.153 \times 10^{-3}$; and the cut-off for HYPT is $1.837 \times 10^{-2}$).

The seven metabolites model and the three metabolites model each represent a formula to determine whether or not a patient is a responder. The p value represents probability of a patient being a responder. When the p value is 0.5 or more, the patient is determined as a responder.

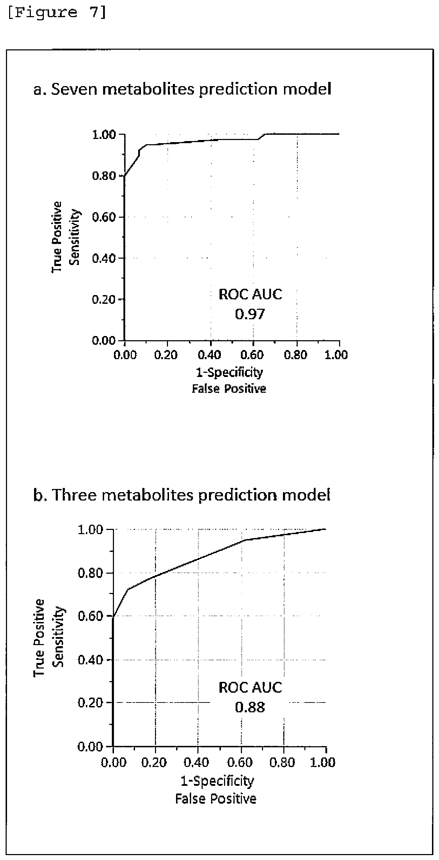

AUC of ROC curve in the seven metabolites model was 0.97 (Figure 7a). The sensitivity, specificity, true positive predictive value and false positive predictive value thereof were 0.949, 0.862, 0.902 and 0.912, respectively.

AUC of ROC curve in the three metabolites model was 0.88 (Figure 7b). The sensitivity, specificity, true positive predictive value and false positive predictive value thereof were 0.769, 0.828, 0.857 and 0.794, respectively.

Of formulas (1) to (3), formula (2) shows higher AUC and higher sensitivity than formulas (1) and (3), so formula (2) can have low probability of incorrectly determining a responder as a non-responder. Thus, it can be said that formula (2) is the most useful model formula for determining sensitivity to an anti-cancer agent.

(c) Prediction performance of OS in seven metabolites model and three metabolites model

To verify the prediction ability for OS of the seven metabolites model and three metabolites model, the patients were divided into the group judged to be responder (R group) and the group judged to be non-responder (N-R group) in accordance with the seven metabolites model or the three metabolites model based on the metabolites before starting the therapy, Kaplan-Meier curves were depicted, and then the difference in OS between the R group and the N-R group was studied. The comparison in OS between the R group and the N-R group was performed by Log-rank test.

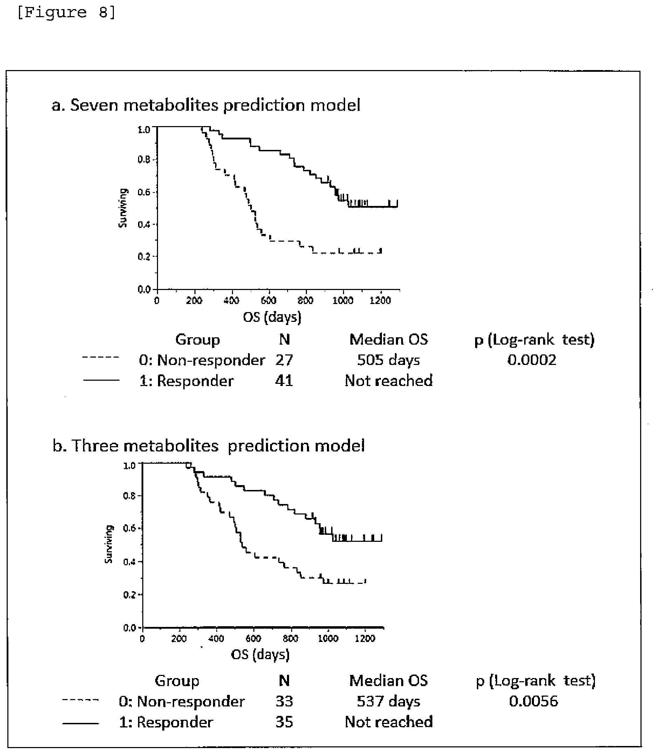

As a result, in both of the seven metabolites model and the three metabolites model, OS was significantly longer in the R group compared to the N-R group (p = 0.0002, p = 0.0056, respectively,), which shows the utility of the present metabolites models (Figure 8a and 8b).

(d) Analysis of OS by COX proportional hazard model for metabolite

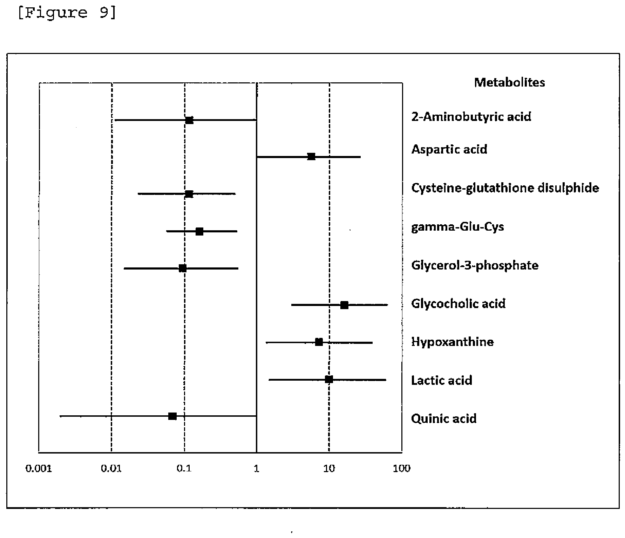

Figure 9 shows hazard ratios and 95% confidence intervals of the metabolites which became significant as a result of the analysis by COX proportional hazard model. It was found that the higher the blood concentrations of 2-aminobutyric acid, CSSG, gamma-Glu-Cys, glycerol-3-phosphate and quinic acid, the longer the survival. It was also found that the higher the blood concentrations of ASP, glycocholic acid, HYPX and lactic acid, the shorter the survival.

Between these survival predictive metabolites and the anti-cancer agent sensitivity predictive metabolites according to (2-2)(a), ASP, CSSG and HYPX are overlapped. Furthermore, as shown in Table 7, the behaviors of ASP, CSSG and HYPX in (2-2)(a) are the same as those in (2-2)(d). Thus, it was shown that these metabolites ASP, CSSG and HYPX are particularly useful.

[Table 7]

| Metabolites | Behavior in (2-2)(a) | Behavior in (2-2)(d) |
|---|---|---|
| ASP | Blood concentration is higher in N-R group than R group. | The higher the blood concentration, the shorter the survival. |
| CSSG | Blood concentration is higher in R group than N-R group. | The higher the blood concentration, the longer the survival. |
| HYPX | Blood concentration is higher in N-R group than R group. | The higher the blood concentration, the shorter the survival. |

(e) Kaplan-Meier curves in each threshold and Log-rank test results of metabolites which became significant in COX proportional hazard model

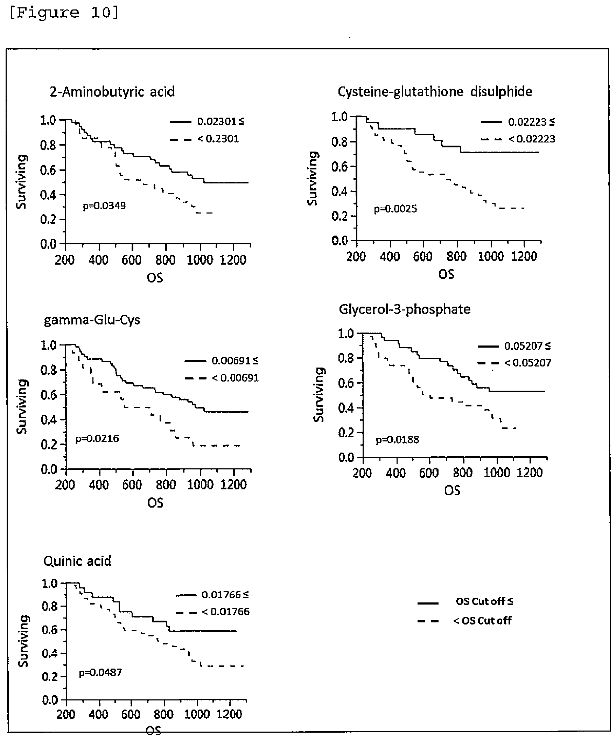

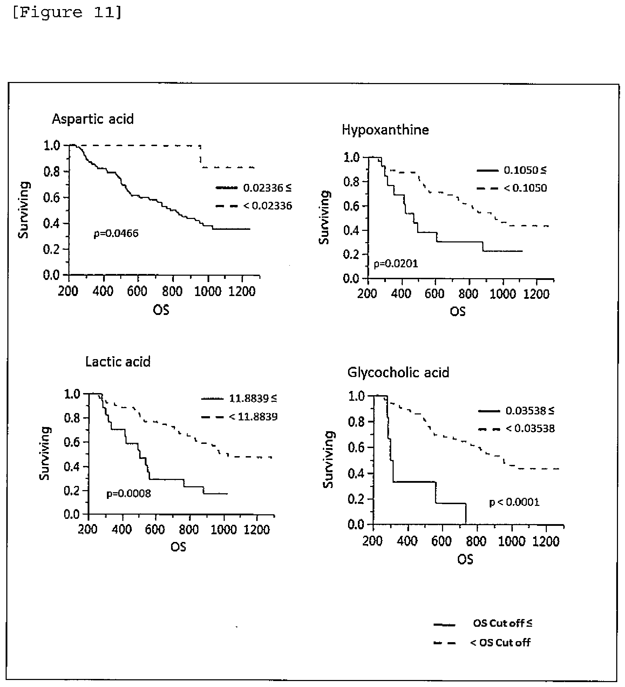

The patients were divided into groups by using appropriate cut-off value for each of the metabolites which became significant as a result of the analysis with the COX proportional hazard model, and then Kaplan-Meier curve was depicted and Log-rank test was performed for each metabolite. Among them, with respect to CSSG and HYPX, the patients were divided into groups with the values same as the respective cut-off values for responder and non-responder. The results of metabolites whose hazard ratio was less than 1 are shown in Figure 10, and the results of metabolites whose hazard ratio was more than 1 are shown in Figure 11. Consequently, significant difference in OS was obtained in all metabolites between the group of equal to or more than the cut-off value and the group of below the cut-off value. This result further shows the usefulness of these metabolites.

**Claims**

1. A marker for determining sensitivity to an anti-cancer agent, the anti-cancer agent including oxaliplatin or a salt thereof, fluorouracil or a salt thereof, and levofolinate or a salt thereof, the marker comprising one or more substances selected from the group consisting of 2DG6P, 2MSE, CSSG, DOPM, GSSG, I4A, P2CB, 1-methyl-2-pyrrolidone, ASP, benzamide, glucaric acid, GL6P, Gly-Gly, HYPT and HYPX.

2. The marker for determining sensitivity to an anti-cancer agent according to claim 1, wherein the anti-cancer agent further includes bevacizumab.

3. A method for determining sensitivity to an anti-cancer agent, the anti-cancer agent including oxaliplatin or a salt thereof, fluorouracil or a salt thereof, and levofolinate or a salt thereof, the method comprising measuring an amount of one or more substances selected from the group consisting of 2DG6P, 2MSE, CSSG, DOPM, GSSG, I4A, P2CB, 1-methyl-2-pyrrolidone, ASP, benzamide, glucaric acid, GL6P, Gly-Gly, HYPT and HYPX in a biological sample from a cancer patient.

4. The method for determining sensitivity according to claim 3, further comprising determining sensitivity of the cancer patient to the anti-cancer agent by comparing a result of the measuring to a control level.

5. The method for determining sensitivity according to claim 4, wherein the control level is a cut-off value for a responder, and the cut-off for 2DG6P is $5.304 \times 10^{-4}\leq$; the cut-off for 2MSE is $1.404 \times 10^{-3}\leq$; the cut-off for CSSG is $2.223 \times 10^{-2}\leq$; the cut-off for DOPM is $1.153 \times 10^{-3}\leq$; the cut-off for GSSG is $1.061 \times 10^{-3}\leq$; the cut-off for I4A is $3.316 \times 10^{-3}\leq$; the cut-off for P2CB is $5.952 \times 10^{-4}\leq$; the cut-off for 1-methyl-2-pyrrolidone is $\leq 8.422 \times 10^{-2}$; the cut-off for ASP is $\leq 3.401 \times 10^{-2}$; the cut-off for benzamide is $\leq 9.859 \times 10^{-2}$; the cut-off for glucaric acid is $\leq 1.058 \times 10^{-3}$; the cut-off for GL6P is $\leq 8.167 \times 10^{-4}$; the cut-off for Gly-Gly is $\leq 5.349 \times 10^{-3}$; the cut-off for HYPT is $\leq 1.837 \times 10^{-2}$; and the cut-off for HYPX is $\leq 1.050 \times 10^{-1}$.

6. The method for determining sensitivity according to claim 3, further comprising determining whether the cancer patient is a responder or not by calculating probability (p) of the cancer patient being the responder with formula (1):

$$p = \frac{1}{1 + e^{-(-3.067 + 19.971 \times (2DG6P) + 2.844 \times (CSSG) + 2.864 \times (HYPT) + 17.68 \times (I4A) + 17.81 \times (P2CB))}} \qquad (1)$$

(wherein, 2DG6P, CSSG, I4A and P2CB represent 1, respectively, when a result of the measuring for each substance is equal to or more than a respective cut-off value, and represent 0, respectively, when the result of the measuring for each substance is less than the respective cut-off value; and HYPT represents 1 when a result of the measuring for HYPT is equal to or less than a cut-off value, and represents 0 when the result of the measuring is more than the cut-off value, and wherein the cut-off for 2DG6P is $5.304 \times 10^{-4}$; the cut-off for CSSG is $2.223 \times 10^{-2}$; the cut-off for I4A is $3.316 \times 10^{-3}$; the cut-off for P2CB is $5.952 \times 10^{-4}$; and the cut-off for HYPT is $1.837 \times 10^{-2}$).

7. The method for determining sensitivity according to claim 3, further comprising determining whether the cancer patient is a responder or not by calculating probability (p) of the cancer patient being the responder with formula (2):

$$p = \frac{1}{1 + e^{(-11.5959 - ((2DG6P) + (2MSE) + (ASP) + (CSSG) + (DOPM) + (GL6P) + (HYPT)))}} \qquad (2)$$

(wherein, 2DG6P represents 10.2190 when a result of the measuring for 2DG6P is equal to or more than a cut-off value, and represents -10.2190 when the result of the measuring is less than the cut-off value; 2MSE represents 1.4778 when a result of the measuring for 2MSE is equal to or more than a cut-off value, and represents -1.4778 when the result of the measuring is less than the cut-off value; ASP represents -1.4976 when a result of the measuring for ASP is equal to or more than a cut-off value, and represents 1.4976 when the result of the measuring is less than the cut-off value; CSSG represents 2.0937 when a result of the measuring for CSSG is equal to or more than a cut-off value, and represents -2.0937 when the result of the measuring is less than the cut-off value; DOPM represents 2.2258 when a result of the measuring for DOPM is equal to or more than a cut-off value, and represents -2.2258 when the result of the measuring is less than the cut-off value; GL6P represents -1.6623 when a result of the measuring for GL6P is equal to or more than a cut-off value, and represents 1.6623 when the result is less than the cut-off value; and HYPT represents -2.3200 when a result of the measuring for HYPT is equal to or more than a cut-off value, and represents 2.3200 when the result of the measuring is less than the cut-off value, and wherein the cut-off for 2DG6P is $5.304 \times 10^{-4}$; the cut-off for 2MSE is $1.404 \times 10^{-3}$; the cut-off for ASP is $3.401 \times 10^{-2}$; the cut-off for CSSG is $2.223 \times 10^{-2}$; the cut-off for DOPM is $1.153 \times 10^{-3}$; the cut-off for GL6P is $8.167 \times 10^{-4}$; and the cut-off for HYPT is $1.837 \times 10^{-2}$).

8. The method for determining sensitivity according to claim 3, further comprising determining whether the cancer patient is a responder or not by calculating probability (p) of the cancer patient being the responder with formula (3):

$$p = \frac{1}{1+e^{(-1.7898-\{(CSSG)+(DOPM)+(HYPT)\})}} \qquad (3)$$

(wherein, CSSG represents 1.8701 when a result of the measuring for CSSG is equal to or more than a cut-off value, and represents -1.8701 when the result of the measuring is less than the cut-off value; DOPM represents 1.4081 when a result of the measuring for DOPM is equal to or more than a cut-off value, and represents -1.4081 when the result of the measuring is less than the cut-off value; and HYPT represents -1.0869 when a result of the measuring for HYPT is equal to or more than a cut-off value, and represents 1.0869 when the result of the measuring is less than the cut-off value, and wherein the cut-off for CSSG is $2.223 \times 10^{-2}$; the cut-off for DOPM is $1.153 \times 10^{-3}$; and the cut-off for HYPT is $1.837 \times 10^{-2}$).

9. The method for determining sensitivity according to any one of claims 3 to 8, wherein the biological sample is a biological sample from a cancer patient to whom the anti-cancer agent has been administered.

10. The method for determining sensitivity according to any one of claims 3 to 9, wherein the anti-cancer agent further includes bevacizumab.

11. A method for determining a total tumor diameter of a cancer patient, the method comprising measuring an amount of one or more substances selected from the group consisting of ASP and CSSG in a biological sample from the cancer patient.

12. A marker for predicting prognosis in a therapy with an anti-cancer agent, the anti-cancer agent including oxaliplatin or a salt thereof, fluorouracil or a salt thereof, and levofolinate or a salt thereof, the marker comprising one or more substances selected from the group consisting of 2-aminobutyric acid, CSSG, gamma-Glu-Cys, glycerol-3-phosphate, quinic acid, ASP, glycocholic acid, HYPX and lactic acid.

13. The marker for predicting prognosis according to claim 12, wherein the anti-cancer agent further includes bevacizumab.

14. A method for predicting prognosis in a therapy with an anti-cancer agent, the anti-cancer agent including oxaliplatin or a salt thereof, fluorouracil or a salt thereof, and levofolinate or a salt thereof, the method comprising measuring an amount of one or more substances selected from the group consisting of 2-aminobutyric acid, CSSG, gamma-Glu-Cys, glycerol-3-phosphate, quinic acid, ASP, glycocholic acid, HYPX and lactic acid in a biological sample from a cancer patient.

15. The method for predicting prognosis according to claim 14, wherein the anti-cancer agent further includes bevacizumab.

16. A kit for performing the method for determining sensitivity according to any one of claims 3 to 11, the kit comprising a protocol for measuring an amount of one or more substances selected from the group consisting of 2DG6P, 2MSE, CSSG, DOPM, GSSG, I4A, P2CB, 1-methyl-2-pyrrolidone, ASP, benzamide, glucaric acid, GL6P, Gly-Gly, HYPT and HYPX in the biological sample from the cancer patient.

17. A kit for performing the method for predicting prognosis according to claim 14 or 15, the kit comprising a protocol for measuring an amount of one or more substances selected from the group consisting of 2-aminobutyric acid, CSSG, gamma-Glu-Cys, glycerol-3-phosphate, quinic acid, ASP, glycocholic acid, HYPX and lactic acid in the biological sample from the cancer patient.

18. A screening method for an anti-cancer agent sensitivity enhancer, the anti-cancer agent including oxaliplatin or a salt thereof, fluorouracil or a salt thereof, and levofolinate or a salt thereof, the method comprising employing, as an index, expression variation of one or more substances selected from the group consisting of 2DG6P, 2MSE, CSSG, DOPM, GSSG, I4A, P2CB, 1-methyl-2-pyrrolidone, ASP, benzamide, glucaric acid, GL6P, Gly-Gly, HYPT, HYPX, 2-aminobutyric acid, gamma-Glu-Cys, glycerol-3-phosphate, quinic acid, glycocholic acid and lactic acid in a cancer cell line or a biological sample from a cancer-bearing animal in the presence of the anti-cancer agent.

19. The screening method according to claim 18, wherein the anti-cancer agent further includes bevacizumab.

20. An anti-cancer agent sensitivity enhancer, the anti-cancer agent including oxaliplatin or a salt thereof, fluorouracil or a salt thereof, and levofolinate or a salt thereof, wherein the anti-cancer agent sensitivity enhancer is obtained by the method according to claim 18 or 19.

21. A composition for cancer therapy, the composition comprising a combination of the anti-cancer agent sensitivity enhancer according to claim 20 with an anti-cancer agent including oxaliplatin or a salt thereof, fluorouracil or a salt thereof, and levofolinate or a salt thereof.

22. The composition for cancer therapy according to claim 21, wherein the anti-cancer agent further includes bevacizumab.

[Figure 1]

[Figure 2]

Baseline Tumor diameter(mm) = 39. 5 + 585.5×Asp                    Baseline Tumor diameter (mm) = 171.1 - 3540.6×CSSG

[Figure 3]

[Figure 4]

4A. PFS

p = 0.0331

CSSG High

CSSG Low

| Group | Median PFS |
|---|---|
| CSSG Low: | 363 days |
| CSSG High: | 425 days |

4B. OS

p = 0.0025

CSSG High

CSSG Low

| Group | Median survival | 1 yeas survival | 2 years survival |
|---|---|---|---|
| CSSG Low: | 736 days | 80.9% | 53.2% |
| CSSG High: | Not reached | 90.5% | 76.2% |

EP 3 605 103 A1

[Figure 5]

31

[Figure 6]

[Figure 7]

a. Seven metabolites prediction model

ROC AUC
0.97

b. Three metabolites prediction model

ROC AUC
0.88

[Figure 8]

a. Seven metabolites prediction model

| Group | N | Median OS | p (Log-rank test) |
|---|---|---|---|
| 0: Non-responder | 27 | 505 days | 0.0002 |
| 1: Responder | 41 | Not reached | |

b. Three metabolites prediction model

| Group | N | Median OS | p (Log-rank test) |
|---|---|---|---|
| 0: Non-responder | 33 | 537 days | 0.0056 |
| 1: Responder | 35 | Not reached | |

[Figure 9]

[Figure 10]

[Figure 11]

## INTERNATIONAL SEARCH REPORT

| International application No. |
| --- |
| PCT/JP2018/013340 |

**A. CLASSIFICATION OF SUBJECT MATTER**

Int.Cl. G01N33/68(2006.01)i, A61K31/282(2006.01)i, A61K31/513(2006.01)i, A61K31/519(2006.01)i, A61K39/395(2006.01)i, A61P35/00(2006.01)i, G01N33/50(2006.01)i

According to International Patent Classification (IPC) or to both national classification and IPC

**B. FIELDS SEARCHED**

Minimum documentation searched (classification system followed by classification symbols)

Int.Cl. G01N33/68, A61K31/282, A61K31/513, A61K31/519, A61K39/395, A61P35/00, G01N33/50

Documentation searched other than minimum documentation to the extent that such documents are included in the fields searched

| | |
| --- | --- |
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

JSTPlus/JMEDPlus/JST7580 (JDreamIII), CAplus/MEDLINE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| A | 隈元謙介ほか, FOLFOX 療法の治療効果予測因子の探索, 日本臨床, 20 April 2015, vol. 73 special issue 4, pp. 530-537, (Nippon rinsho), non-official translation (KUMAMOTO, Kensuke et al., "Search for predictive factors of therapeutic effect in FOLFOX treatment") | 1-10, 16, 18-19 (parts) |
| A | 永井慎一郎ほか, "Predictive biomarkers of mFOLFOX6 efficacy in colorectal cancer", 日本外科学会雑誌, 2011, vol. 112, special extra edition (1・2), p. 666, (NAGAI, Shinichiro et al., Journal of Japan Surgical Society) | 1-10, 16, 18-19 (parts) |
| A | 吉村麻衣子ほか, OCT2/TS 二重免疫染色による大腸癌 FOLFOX 療法の効果予測, 日本病理学会会誌, 12 April 2016, vol. 105, no. 1, p. 487, (Transactiones Societatis Pathologicae Japonicae), non-official translation (YOSHIMURA, Maiko et al., "Prediction of effect on FOLFOX treatment for colorectal cancer by OCT2/TS double immunostaining") | 1-10, 16, 18-19 (parts) |

☐ Further documents are listed in the continuation of Box C.     ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 25 June 2018 (25.06.2018) | 03 July 2018 (03.07.2018) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2018/013340 |

---

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 20-22
because they relate to parts of the international application that do not comply with the prescribed requirements to such an
extent that no meaningful international search can be carried out, specifically:

Claims 20-22 pertain to an anticancer agent-sensitive agonist and a
cancer treatment composition obtained by combining the anticancer agonist and
an anticancer agent. However, since specific compounds for the anticancer
agent and the agonist are not mentioned at all in the specification, and it
is impossible to presume what can be composed of the anticancer agonist, a
meaningful international search could not be carried out.

3. ☐ Claims Nos.:
because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable
claims.

2. ☐ As all searchable claims could be searched without effort justifying additional fees, this Authority did not invite payment of
additional fees.

3. ☐ As only some of the required additional search fees were timely paid by the applicant, this international search report covers
only those claims for which fees were paid, specifically claims Nos.:

4. ☒ No required additional search fees were timely paid by the applicant.   Consequently, this international search report is
restricted to the invention first mentioned in the claims; it is covered by claims Nos.:   1-10, 16, 18-19 (parts)

**Remark on Protest**

☐ The additional search fees were accompanied by the applicant's protest and, where applicable, the
payment of a protest fee.

☐ The additional search fees were accompanied by the applicant's protest but the applicable protest
fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

| International application No. |
|---|
| PCT/JP2018/013340 |

Box III

The inventions in claims 1-19 are clearly divided into three separate invention groups: an invention pertaining to a marker for determining the susceptibility of an anticancer agent (hereinafter abbreviated as an "anticancer agent") comprising oxaliplatin or a salt thereof, fluorouracil or a salt thereof, and levofolinate or a salt thereof; an invention pertaining to a method for judging the sum of the tumor diameters of a cancer patient; and an invention pertaining to a prognosis prediction marker at the time of treatment with an anticancer agent. In addition, since the technical features of the marker for determining the sensitivity of an anticancer agent and the prognosis prediction marker at the time of treatment with an anticancer agent do not make a contribution over the prior art in light of the disclosure of document 1, said features cannot be said to be a special technical feature. Accordingly, the claims are classified into 25 inventions having the following special technical features, respectively.

(Invention 1) claims 1-10, 16, and 18-19 (parts)
Invention pertaining to 2DG6P as a marker for determining the sensitivity of an anticancer agent.
(Invention 2) Claims 1-10, 16, and 18-19 (parts)
Invention pertaining to 2MSE as a marker for determining the sensitivity of an anticancer agent.
(Invention 3) Claims 1-10, 16, and 18-19 (parts)
Invention pertaining to CSSG as a marker for determining the sensitivity of an anticancer agent.
(Invention 4) Claims 1-10, 16, and 18-19 (parts)
Invention pertaining to DOPM as a marker for determining the sensitivity of an anticancer agent.
(Invention 5) Claims 1-10, 16, and 18-19 (parts)
Invention pertaining to CSSG as a marker for determining the sensitivity of an anticancer agent.
(Invention 6) Claims 1-10, 16, and 18-19 (parts)
Invention pertaining to I4A as a marker for determining the sensitivity of an anticancer agent.
(Invention 7) Claims 1-10, 16, and 18-19 (parts)
Invention pertaining to P2CM as a marker for determining the sensitivity of an anticancer agent.
(Invention 8) Claims 1-10, 16, and 18-19 (parts)
Invention pertaining to 1-methyl-2-pyrrolidone as a marker for determining the sensitivity of an anticancer agent.
(Invention 9) Claims 1-10, 16, and 18-19 (parts)
Invention pertaining to ASP as a marker for determining the sensitivity of an anticancer agent.
(Invention 10) Claims 1-10, 16, and 18-19 (parts)
Invention pertaining to benzamide as a marker for determining the sensitivity of an anticancer agent.
(Invention 11) Claims 1-10, 16, and 18-19 (parts)
Invention pertaining to glucaric acid as a marker for determining the sensitivity of an anticancer agent.
(Invention 12) Claims 1-10, 16, and 18-19 (parts)
Invention pertaining to GL6P as a marker for determining the sensitivity of an anticancer agent.
(Invention 13) Claims 1-10, 16, and 18-19 (parts)
Invention pertaining to Gly-Gly as a marker for determining the sensitivity of an anticancer agent.
(Invention 14) Claims 1-10, 16, and 18-19 (parts)
Invention pertaining to HYPT as a marker for determining the sensitivity of an anticancer agent.

Form PCT/ISA/210 (extra sheet) (January 2015)

**INTERNATIONAL SEARCH REPORT**

International application No.

PCT/JP2018/013340

(Invention 15) Claims 1-10, 16, and 18-19 (parts)
Invention pertaining to HYPX as a marker for determining the sensitivity of an anticancer agent.
(Invention 16) Claims 11, and 16 (part)
Invention pertaining to a method for determining the sum of the tumor diameters of a cancer patient.
(Invention 17) Claims 12-15, and 17 (part)
Invention pertaining to 2-aminobutyric acid as a prognosis prediction marker.
(Invention 18) Claims 12-15, and 17 (part)
Invention pertaining to CSSG as a prognosis prediction marker.
(Invention 19) Claims 12-15, and 17 (part)
Invention pertaining to gamma-Glu-Cys as a prognosis prediction marker.
(Invention 20) Claims 12-15, and 17 (part)
Invention pertaining to glycerol-3-phosphate as a prognosis prediction marker.
(Invention 21) Claims 12-15, and 17 (part)
Invention pertaining to quinic acid as a prognosis prediction marker.
(Invention 22) Claims 12-15, and 17 (part)
Invention pertaining to ASP as a prognosis prediction marker.
(Invention 23) Claims 12-15, and 17 (part)
Invention pertaining to glycocholic acid as a prognosis prediction marker.
(Invention 24) Claims 12-15, and 17 (part)
Invention pertaining to HYPX as a prognosis prediction marker.
(Invention 25) Claims 12-15, and 17 (part)
Invention pertaining to lactic acid as a prognosis prediction marker.

Claim 20-22 pertain to a cancer treatment composition comprising an anticancer agent-sensitive agonist and a combination of the anticancer agent and an anticancer agent, but the description does not mention any specific compounds for the anticancer agent-sensitive agonist at all, and a meaningful international search cannot be carried out because it is impossible to presume what substance can be composed of the anticancer agent-sensitive agonist.

Document 1: 隈元謙介ほか, FOLFOX 療法の治療効果予測因子の探索, 日本臨床, 20 April 2015, vol. 73 special issue 4, pp. 530-537, (Nippon rinsho), non-official translation (KUMAMOTO, Kensuke et al., "Search for predictive factors of therapeutic effect in FOLFOX treatment")

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009096189 A
- WO 2011052750 A
- WO 2012127984 A
- WO 2013125675 A
- WO 2013125509 A

- JP 2017007980 A
- JP 2017014167 A
- JP 2006504093 A
- JP 2016153808 A

**Non-patent literature cited in the description**

- *J Natl Cancer Inst.,* 02 February 2000, vol. 92 (3), 205-16
- *J Proteome Res.,* September 2003, vol. 2 (5), 488-94

- *Metabolomics.,* March 2010, vol. 6 (1), 78-95
- *Metabolomics.,* April 2013, vol. 9 (2), 444-453
- *Journal of the Royal Statistical Society,* vol. 57, 289-300